# EUROPEAN PATENT APPLICATION

(11) **EP 4 725 470 A1**
(43) Date of publication of application: **15.04.2026**
(21) Application number: 24819431.8
(22) Date of filing: 07.06.2024
(51) Int. Cl.: A61K 6/30, A61K 6/62, A61K 6/76, A61K 6/77

(54) **CURABLE DENTAL COMPOSITION**

(30) Priority: 08.06.2023 JP 2023095102
(71) Applicant: Kuraray Noritake Dental Inc., Kurashiki-shi, Okayama 710-0801 (JP)
(72) Inventor: ATAKA, Kensuke, Tainai-shi, Niigata 959-2653 (JP); NOJIRI, Yamato, Tokyo 100-0004 (JP)
(74) Representative: D Young & Co LLP
(86) International application number: PCT/JP2024/020946
(87) International publication number: WO 2024/253204

(57) **Abstract**

The present invention provides a curable dental composition that exhibits adequate flowability to reduce lifting of prostheses during luting while achieving excellent removability of excess cement at the time of preliminary irradiation. The present invention relates to a curable dental composition comprising a polymerizable monomer (A), a filler (B), a polymerization initiator (C), a polymerization accelerator (D), and a rheology modifier (E), wherein: the polymerization initiator (C) comprises a photopolymerization initiator (C-1), the curable dental composition exhibits a viscosity V1 of 10,000 Pa·s or more and less than 20,000 Pa·s at an initial shear rate D1 (0.01 s⁻¹) when measured for viscosity over a total time period of 3 minutes using a rotational rheometer, with 1 minute at shear rate D1 (0.01 s⁻¹), followed by 1 minute at shear rate D2 (10 s⁻¹), and then 1 minute at shear rate D1 again, the curable dental composition exhibits a viscosity V2 of 100 Pa·s or less at shear rate D2 (10 s⁻¹), and the time T required for viscosity V3 to reach 10,000 Pa·s after the shear rate is switched from D2 back to D1 is less than 40 seconds from when the shear rate is switched from D2 to D1.

## Description

### TECHNICAL FIELD

The present invention relates to a curable dental composition used in dental treatments for procedures such as luting of dental prostheses, such as crowns, inlays, and bridges, to tooth structure.

More specifically, the present invention relates to a curable dental composition (preferably, a dental cement) that exhibits excellent flowability when luting tooth structure and dental prostheses, and offers superior removability when excess cement protruding from the margin area is removed in a semi-cured state after preliminary irradiation with a photoirradiator.

### BACKGROUND ART

In the restorative treatment of tooth structures (enamel, dentin, and cementum) damaged by caries or other conditions, dental cement is used as a material for luting dental prostheses, such as crowns, inlays, and bridges, to the lost crown portion.

Generally, dental cement is composed of a polymerizable monomer, a filler, and a polymerization initiator.

When luting a dental prosthesis to tooth structure using a dental cement, an excess of dental cement is applied to the inner wall surface of the dental prosthesis before pressing it against the tooth structure. For this pressing procedure, dental cement is typically applied in excess to prevent detachment of the dental prosthesis.

Excess dental cement protrudes from the contact area between the tooth structure and the dental prosthesis. Desirably, the protruding portion of dental cement (hereinafter, referred to as "excess cement") should be completely removed because it is not only aesthetically poor but may also damage the oral tissue once it cures.

Excess cement is usually removed using a dental probe or similar tool. However, the removal of excess cement with a dental probe is difficult to achieve when the excess cement is entirely uncured. As such, the excess cement is first cured by exposing it to light before removal.

Conversely, once the excess cement is completely cured, it adheres to the tooth structure and becomes extremely difficult to remove. For this reason, in clinical settings such as dental practices, the excess cement is removed after it has cured to some degree and its flowability has been reduced (semi-cured state). To achieve this semi-cured state, the excess cement is exposed to light for a few seconds (typically around 1 to 5 seconds). This exposure to light is referred to herein as "preliminary irradiation."

Once the excess cement is removed after preliminary irradiation, a second light exposure is applied to fully cure the cement at the fitting area. This second exposure is referred to herein as "main irradiation." The duration of the main irradiation depends on the type of light source and the intensity of light adopted by the photoirradiator used, and is generally set to about 10 to 20 seconds.

Desirably, dental cement should have high flowability because issues such as detachment of the prosthesis or the development of secondary caries may occur after restoration when the prosthesis lifts during luting and compromises the fit between the crown portion and the prosthesis.

On the other hand, excess cement spreads thinly under its own weight if the dental cement overflowing from the fitting area is highly flowable. This spreading of excess cement increases the area that must be removed, and the excess cement, particularly in the thin regions, becomes fully cured during preliminary irradiation, making removal extremely difficult. To prevent this, it is desirable for the excess cement to have high shape retention.

One method to improve the removability of excess dental cement is described in, for example, Patent Literature 1, which teaches that a specific polymerization initiator system can be used to obtain a curable dental composition that allows for excellent removal in a semi-cured state after a brief preliminary irradiation of 1 to 2 seconds using a photoirradiator while also achieving superior mechanical characteristics both immediately after main irradiation and after one day.

Patent Literature 2 describes a dental cement in which the distance of the plastic flow of the uncured kneaded material is 2 mm or less. It is stated that this imparts shape retention to the kneaded material, preventing it from sagging and flowing under its own weight, thereby achieving excellent removability of excess cement after curing.

Patent Literature 3 discloses a dental cement kit where the onset time of polymerization upon contact between a primer and dental cement is maintained within a certain range. It is stated that this equalizes the curability between the area where the primer and dental cement are in contact-specifically, the central portion of the excess cement-and the preliminarily irradiated surface of the excess cement, allowing for easy removal of the excess cement in one piece.

### CITATION LIST

### Patent Literature

Patent Literature 1: JP 2014-111555 A
Patent Literature 2: JP 2019-167334 A
Patent Literature 3: WO2020/111142

### SUMMARY OF INVENTION

### Technical Problem

However, in the curable dental composition described in Patent Literature 1, further examination is needed with respect to the flowability of excess cement.

Particularly, there are areas for improvement with respect to aspects such as an examination of the ease of removing thin film portions in situations involving sagging and spreading of excess cement.

The dental cement described in Patent Literature 2 is a dental glass ionomer cement composition for luting that lacks a photoinitiator, and it is not intended to improve the ease of removing excess cement when removing it immediately after preliminary irradiation.

Furthermore, as examined by the present inventors, while the dental cement kit described in Patent Literature 3 considers the flowability of water-based dental adhesive composition (A) and improves the removability of excess cement when used as a dental cement kit, the dental cement kit focuses on curability control as a whole, and the interaction between water-based dental adhesive composition (A) and curable dental composition (B) is of significant importance.

As such, no consideration is given to issues arising when the curable dental composition (B) is used alone, including issues involving increased flowability of dental cement and thinning of excess cement when the curable dental composition (B) is used by itself.

Accordingly, in embodiments using the curable dental composition alone, there remain areas for improvement, including an examination of the ease of removing thin film portions involving sagging and spreading of excess cement.

It is accordingly an object of the present invention to provide a curable dental composition that exhibits adequate flowability to reduce lifting of prostheses during luting while achieving excellent removability of excess cement at the time of preliminary irradiation.

### Solution to Problem

The present inventors conducted intensive studies to find a solution to the issues discussed above, and successfully created a dental cement that possesses two contradictory properties: high flowability while luting dental prostheses and easy removability of excess cement due to its high shape retention. This was made possible by a composition that exhibits unique viscosity behavior-a large decrease in viscosity under applied pressure compared to when at rest, and a rapid return to its resting viscosity upon relieving the stress. Building upon this finding, the present inventors conducted additional examinations, ultimately resulting in the completion of the present invention.

Specifically, the present invention includes the following.
[1] A curable dental composition comprising a polymerizable monomer (A), a filler (B) with an average particle diameter of 1 µm or more, a polymerization initiator (C), a polymerization accelerator (D), and a rheology modifier (E), wherein:
   the polymerization initiator (C) comprises a photopolymerization initiator (C-1),
   the curable dental composition exhibits a viscosity V1 of 10,000 Pa·s or more and less than 20,000 Pa·s at an initial shear rate D1 (0.01 s⁻¹) when measured for viscosity over a total time period of 3 minutes using a rotational rheometer, with 1 minute at shear rate D1 (0.01 s⁻¹), followed by 1 minute at shear rate D2 (10 s⁻¹), and then 1 minute at shear rate D1 again,
   the curable dental composition exhibits a viscosity V2 of 100 Pa·s or less at shear rate D2 (10 s⁻¹), and
   the time T required for viscosity V3 to reach 10,000 Pa·s after the shear rate is switched from D2 back to D1 is less than 40 seconds from when the shear rate is switched from D2 to D1.
[2] The curable dental composition according to [1], wherein the polymerizable monomer (A) comprises 0 parts or more by mass and less than 15 parts by mass of a hydrophilic polymerizable monomer having no acidic group (A-1a), and 55 parts or more by mass of a hydrophobic polymerizable monomer having no acidic group (A-1b), within total 100 parts by mass of the polymerizable monomer (A).
[3] The curable dental composition according to [1] or [2], wherein the filler (B) is hydrophobized with a hydrophobizing agent (x-1).
[4] The curable dental composition according to [1] or [2], wherein the hydrophobizing agent (x-1) comprises a silane coupling agent that has a polymerizable group and is represented by the following general formula (1),

   Y-SiRₙX₍₃₋ₙ₎ (1)

   wherein Y represents a polymerizable group, or a monovalent organic group having a polymerizable group, R represents a group selected from the group consisting of an alkyl group, an aryl group, and an aralkyl group, X represents a hydroxyl group or a hydrolyzable group, and n represents an integer of 0, 1, or 2, where R may be the same or different from each other when R is plural, and X may be the same or different from each other when X is plural.
[5] The curable dental composition according to [4], wherein Y in the general formula (1) is a group resulting from the bonding of a (meth)acryloyl group and an organic group via a divalent group containing an oxygen atom, and the organic group is an alkyl group having 5 to 11 carbon atoms.
[6] The curable dental composition according to any one of [1] to [5], wherein the polymerization initiator (C) comprises a chemical polymerization initiator (C-2).
[7] The curable dental composition according to any one of [1] to [6], wherein the polymerization accelerator (D) comprises a chemical polymerization accelerator (D-2).
[8] The curable dental composition according to [7], which comprises a first agent and a second agent, wherein:
   the first agent comprises the polymerizable monomer (A), the filler (B), and the chemical polymerization initiator (C-2),
   the second agent comprises the polymerizable monomer (A), the filler (B), and the chemical polymerization accelerator (D-2), and
   the first agent and/or the second agent comprise the rheology modifier (E).
[9] The curable dental composition according to any one of [1] to [8], wherein the rheology modifier (E) comprises an inorganic fine particle (E-1) having an average particle diameter of less than 1 µm.
[10] The curable dental composition according to [9], wherein the inorganic fine particle (E-1) comprises a spherical inorganic fine particle (E-1a).
[11] The curable dental composition according to [10], wherein the spherical inorganic fine particle (E-1a) is hydrophobized with a hydrophobizing agent (x-1).
[12] The curable dental composition according to [11], wherein the hydrophobizing agent (x-1) is hydrophobized with a silane coupling agent that has a polymerizable group and is represented by the following general formula (1),

   Y-SiRₙX₍₃₋ₙ₎ (1)

   wherein Y represents a polymerizable group, or a monovalent organic group having a polymerizable group, R represents a group selected from the group consisting of an alkyl group, an aryl group, and an aralkyl group, X represents a hydroxyl group or a hydrolyzable group, and n represents an integer of 0, 1, or 2, where R may be the same or different from each other when R is plural, and X may be the same or different from each other when X is plural.
[13] A dental cement comprising a curable dental composition of any one of [1] to [12].

### Advantageous Effects of Invention

According to the present invention, a curable dental composition can be provided that exhibits adequate flowability to reduce lifting of prostheses during luting while achieving excellent removability of excess cement at the time of preliminary irradiation.

According to the present invention, the curable dental composition does not become excessively thin as it spreads as excess cement during luting, enabling prompt removal of the excess cured product by preliminary irradiation.

### BRIEF DESCRIPTION OF DRAWINGS

[FIG. 1] A schematic view representing usage of a curable dental composition of the present invention.
[FIG. 2] A schematic view representing an example of changes in shear rate and viscosity.

### DESCRIPTION OF EMBODIMENTS

A curable dental composition of the present invention comprises a polymerizable monomer (A), a filler (B) with an average particle diameter of 1 µm or more, a polymerization initiator (C), a polymerization accelerator (D), and a rheology modifier (E), wherein:
the polymerization initiator (C) comprises a photopolymerization initiator (C-1),
the curable dental composition exhibits a viscosity V1 of 10,000 Pa·s or more and less than 20,000 Pa·s at an initial shear rate D1 (0.01 s⁻¹) when measured for viscosity over a total time period of 3 minutes using a rotational rheometer, with 1 minute at shear rate D1 (0.01 s⁻¹), followed by 1 minute at shear rate D2 (10 s⁻¹), and then 1 minute at shear rate D1 again,
the curable dental composition exhibits a viscosity V2 of 100 Pa·s or less at shear rate D2 (10 s⁻¹), and
the time T required for viscosity V3 to reach 10,000 Pa·s after the shear rate is switched from D2 back to D1 is less than 40 seconds from when the shear rate is switched from D2 to D1.

With the configuration of the present invention, the viscosity greatly decreases under applied pressure relative to its value at rest, and quickly returns to the resting viscosity upon relieving the stress. While the exact reason for this unique viscosity behavior and the resulting excellent effects remains somewhat unclear, it is speculated that this arises from the ability to simultaneously exhibit the following physical properties.

A dental cement that exhibits a viscosity V2 of 100 Pa·s or less at a shear rate D2 undergoes sufficient viscosity decrease under the pressure generated when bonding a dental prosthesis to a tooth. This prevents the dental prosthesis from lifting and resulting in a poor fit.

Furthermore, in such a cement material, because the time T required for viscosity V3 to rise to 10,000 Pa·s after switching the shear rate from D2 to D1 is less than 40 seconds, the excess cement does not sag and spread under its own weight, preventing issues where thinly spread, sagged excess cement becomes undetachable after being cured by preliminary irradiation. Moreover, because a certain minimum thickness is ensured for the excess cement, the excess cement can be prevented from curing excessively during preliminary irradiation and becoming difficult to remove.

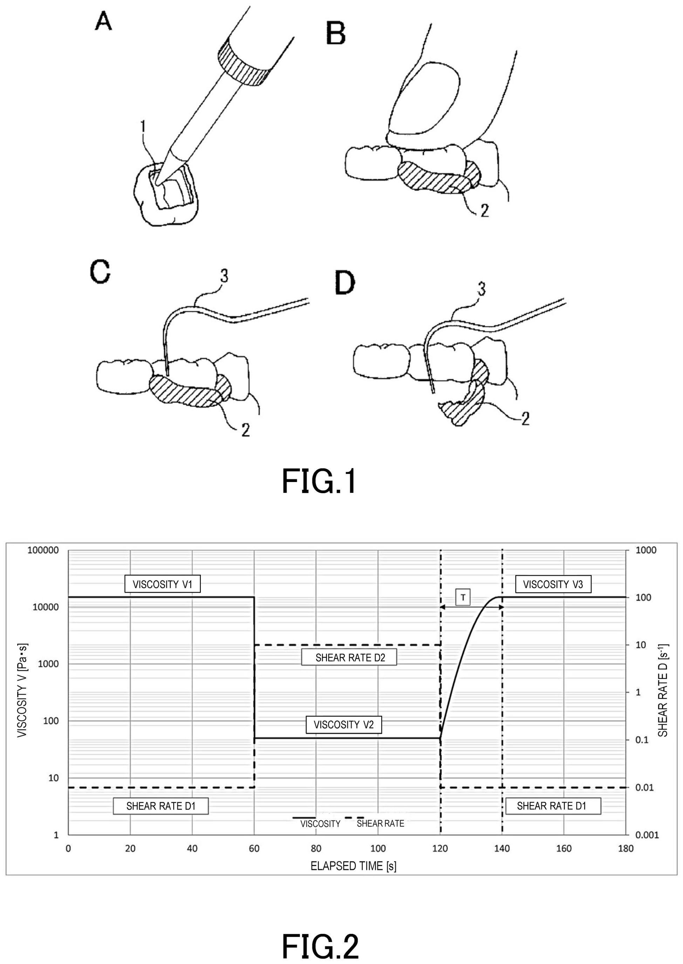

In dental cement applications, as illustrated in FIG. 1A, an adequate amount of uncured dental cement 1 (curable dental composition) is first injected into the underside (recessed portion) of the dental prosthesis to enable luting. Here, the uncured dental cement 1 (curable dental composition) is injected in excess to ensure that the dental prosthesis does not detach when fitted onto the patient's tooth.

Subsequently, as illustrated in FIG. 1B, the practitioner (for example, a dentist) aligns and fits the dental prosthesis to the patient's tooth, causing the excess curable dental composition 1 to flow out as excess cement 2 from the space between the dental prosthesis and the tooth.

Following this, the excess cement 2 is cured by preliminary irradiation (typically, exposed to light for about 1 to 5 seconds).

As illustrated in FIG. 1C and FIG. 1D, the cured excess cement 2 is then detached using a dental probe 3, and the separated excess cement 2 is removed.

In the state illustrated in FIG. 1A, which corresponds to shear rate D1, the curable dental composition experiences no applied force, and, as a result, possesses high shape retention and exhibits low flowability.

In contrast, in the state illustrated in FIG. 1B corresponding to shear rate D2, the practitioner applies force, with or without the use of dental instruments. Under this applied pressure, the curable dental composition between the dental prosthesis and the patient's tooth enters a highly flowable state.

In the state illustrated in FIG. 1C and FIG. 1D, which corresponds to shear rate D1, the curable dental composition is already fitted to the patient's tooth. What is notable here is that the excess portion (excess cement 2) protruding from the space between the dental prosthesis and the patient's tooth is under no applied force.

Here, even with the protruding excess portion exhibiting high shape retention and making a transition toward low flowability, a prolonged delay before reaching this state (original state) causes the curable dental composition to sag and spread thinly in the oral cavity due to high flowability, hindering easy removal of the excess portion.

In contrast, a curable dental composition according to the present invention exhibits unique viscosity behavior where the viscosity greatly decreases and enhances flowability under applied pressure (under applied force) compared to when at rest (under no applied force), and quickly returns to the resting viscosity upon relieving the stress. By adjusting the time required for the viscosity to shift from a low-viscosity to a high-viscosity state, it is possible to reduce the incidence of the issue where the cured product becomes undetachable following preliminary irradiation when the curable dental composition spreads thinly due to high flowability, thus enabling easy removal of the excess portion. That is, the composition can achieve both the high flowability necessary during application and the shape retention needed for removal of the excess portion.

Some conventional techniques have focused on curability as in Patent Literature 3. While Patent Literature 3 allows for removal of excess cement, it does not address the flowability of dental cement when used on its own.

That is, there is no technique that is known to have achieved both flowability and easy removability of the excess portion-a property necessary in dental applications for prompt removal of the excess portion following preliminary irradiation-by not only altering viscosity behavior but also controlling the timing of its change, as provided by the present invention.

The curable dental composition exhibits a viscosity V1 of 10,000 Pa·s or more and less than 20,000 Pa·s at an initial shear rate D1 (0.01 s⁻¹) when measured for viscosity over a total time period of 3 minutes using a rotational rheometer, with 1 minute at shear rate D1 (0.01 s⁻¹), followed by 1 minute at shear rate D2 (10 s⁻¹), and then 1 minute at shear rate D1 again,

The viscosity V1 is preferably 10,000 Pa·s or more, more preferably 10,500 Pa·s or more, even more preferably 11,000 Pa·s or more. The viscosity V1 is preferably 20,000 Pa·s or less, more preferably 19,500 Pa·s or less, even more preferably 19,000 Pa·s or less.

The rotational rheometer may be a known rotational rheometer (for example, AR2000 manufactured by TA Instruments Japan Inc. under this trade name).

Changes in shear rate, from D1 to D2 and back to D1, during the viscosity measurement over a total time period of 3 minutes take place as indicated by the dashed lines in FIG. 2.

The viscosity V2 measured at a shear rate D2 (10 s⁻¹) in this viscosity measurement conducted over a total time period of 3 minutes is preferably 99 Pa·s or less, more preferably 80 Pa·s or less, even more preferably 70 Pa·s or less.

The time T required for viscosity V3 to reach 10,000 Pa·s after the shear rate is switched from D2 back to D1 in the viscosity measurement conducted over a total time period of 3 minutes is preferably 35 seconds or less, more preferably 30 seconds or less, even more preferably 25 seconds or less.

With time T falling within these ranges, the viscosity can quickly return to the resting viscosity, making it possible to reduce the incidence of the issue where the cured product becomes undetachable following preliminary irradiation when the curable dental composition spreads thinly due to high flowability, thus enabling easy removal of the excess portion.

### [Polymerizable Monomer (A)]

Preferred for use as the polymerizable monomer (A) used in a curable dental composition of the present invention are radical polymerizable monomers. Examples of radical polymerizable monomers as polymerizable monomer (A) include (meth)acrylate polymerizable monomers, (meth)acrylamide polymerizable monomers, esters, vinyl esters, and vinyl ethers of acids such as α-cyanoacrylic acid, (meth)acrylic acid, α-halogenated acrylic acid, crotonic acid, cinnamic acid, sorbic acid, maleic acid, and itaconic acid, as well as mono-N-vinyl derivatives and styrene derivatives. In view of curability, preferred among these are (meth)acrylate polymerizable monomers, and (meth)acrylamide polymerizable monomers. The polymerizable monomer (A) is broadly classified into polymerizable monomer having no acidic group (A-1), and polymerizable monomer having an acidic group (A-2).

As used herein, "(meth)acryl" is a collective term for "methacryl" and "acryl."

In a curable dental composition of the present invention, when the content of polymerizable monomer (A) is 60 mass% or more in total 100 mass% of the composition, the composition viscosity becomes considerably low, making it difficult to adjust the flowability of the composition to the appropriate range even with the addition of rheology modifier (E). When the content of the polymerizable monomer (A) in total 100 mass% of the composition is less than 10 mass%, the composition viscosity becomes notably high, also leading to difficulty adjusting the flowability of the composition to the appropriate range even with the addition of rheology modifier (E).

Accordingly, the content of the polymerizable monomer (A) in a curable dental composition of the present invention is preferably 10 mass% or more and less than 60 mass%, more preferably 20 mass% or more and less than 50 mass%, even more preferably 25 mass% or more and less than 45 mass% in total 100 mass% of the composition.

### <Polymerizable Monomer Having no Acidic Group (A-1)>

In the present invention, the polymerizable monomer having no acidic group (A-1) can be classified into hydrophilic polymerizable monomer having no acidic group (A-1a) that has a solubility of 4.5 g/L or more in 25°C water, and hydrophobic polymerizable monomer having no acidic group (A-1b) that has a solubility of less than 4.5 g/L in 25°C water.

### · Hydrophilic Polymerizable Monomer Having no Acidic Group (A-1a)

In a curable dental composition of the present invention, the polymerizable monomer (A) comprises preferably less than 15 parts by mass of hydrophilic polymerizable monomer having no acidic group (A-1a) (hereinafter, also referred to simply as "hydrophilic polymerizable monomer (A-1a)"). The hydrophilic polymerizable monomer (A-1a) refers to monomers that do not have acidic groups and have a solubility of 4.5 g/L or more in 25°C water.

In a curable dental composition of the present invention, when the content of hydrophilic polymerizable monomer (A-1a) is 15 parts or more by mass in total 100 parts by mass of polymerizable monomer (A), the hydrophilic interaction with rheology modifier (E) increases, posing difficulty adjusting the flowability with rheology modifier (E).

Accordingly, the content of the hydrophilic polymerizable monomer (A-1a) in a curable dental composition of the present invention is preferably less than 15 parts by mass, more preferably less than 10 parts by mass, even more preferably less than 5 parts by mass in total 100 parts by mass of polymerizable monomer (A). The content of hydrophilic polymerizable monomer (A-1a) may be 0 parts by mass in total 100 parts by mass of polymerizable monomer (A).

The hydrophilic polymerizable monomer (A-1a) improves the wettability of the curable dental composition to tooth structure. Preferred as hydrophilic polymerizable monomer (A-1a) are radical polymerizable monomers lacking acidic groups and having polymerizable groups. In view of ease of radical polymerization, the polymerizable groups are preferably (meth)acryl groups and/or (meth)acrylamide groups. Examples of the hydrophilic polymerizable monomer (A-1a) include:
hydrophilic (meth)acrylate monofunctional polymerizable monomers such as 2-hydroxyethyl methacrylate (commonly known as HEMA), 3-hydroxypropyl (meth)acrylate, 2-hydroxypropyl (meth)acrylate, 1,3-dihydroxypropyl (meth)acrylate, 2,3-dihydroxypropyl (meth)acrylate, 2-((meth)acryloyloxy)ethyltrimethylammonium chloride, and tetrahydrofurfuryl (meth)acrylate;
hydrophilic (meth)acrylate polyfunctional polymerizable monomers such as triethylene glycol dimethacrylate (commonly known as 3G), tetraethylene glycol di(meth)acrylate, nonaethylene glycol di(meth)acrylate, 1,3-bis(methacryloyloxy)-2-propanol, and 1,2-bis(3-methacryloyloxy-2-hydroxypropoxy)ethane (commonly known as #801); and
hydrophilic (meth)acrylamide polymerizable monomers such as N-methylol(meth)acrylamide, N-hydroxyethyl(meth)acrylamide, N,N-bis(2-hydroxyethyl)(meth)acrylamide, N-methoxymethyl(meth)acrylamide, N-ethoxymethyl(meth)acrylamide, diacetone(meth)acrylamide, 4-(meth)acryloylmorpholine, N-trihydroxymethyl-N-methyl(meth)acrylamide, N,N-dimethylacrylamide, N,N-diethylacrylamide, and N-methacryloyloxyethylacrylamide.

In view of wettability to tooth structure and curability, preferred among these hydrophilic polymerizable monomers (A-1a) are HEMA, 3G, and #801.

The hydrophilic polymerizable monomer (A-1a) may be used alone, or two or more thereof may be used in combination.

### · Hydrophobic Polymerizable Monomer Having no Acidic Group (A-1b)

In a curable dental composition of the present invention, the polymerizable monomer (A) preferably comprises a hydrophobic polymerizable monomer having no acidic group (A-1b) (hereinafter, also referred to simply as "hydrophobic polymerizable monomer (A-1b)"). Here, the hydrophobic polymerizable monomer (A-1b) refers to monomers that do not have acidic groups and have a solubility of less than 4.5 g/L in 25°C water. By using the hydrophobic polymerizable monomer (A-1b), the paste flowability can improve when combined with a filler (B) that is hydrophobically treated. The hydrophobic polymerizable monomer (A-1b) can also mitigate the stringiness (stringing tendency) of the curable dental composition, enhancing the mechanical strength of the cured product.

Preferred as hydrophobic polymerizable monomer (A-1b) are radical polymerizable monomers lacking acidic groups and having polymerizable groups. In view of ease of radical polymerization, the polymerizable groups are preferably (meth)acryl groups and/or (meth)acrylamide groups. Examples of the hydrophobic polymerizable monomer (A-1b) include hydrophobic monofunctional polymerizable monomers, and crosslinkable polymerizable monomers such as hydrophobic aromatic bifunctional polymerizable monomers, hydrophobic aliphatic bifunctional polymerizable monomers, and hydrophobic tri- and higher-functional polymerizable monomers.

Examples of the hydrophobic monofunctional polymerizable monomers include methyl (meth)acrylate, ethyl (meth)acrylate, butyl (meth)acrylate, benzyl (meth)acrylate, isobornyl (meth)acrylate, p-cumyl-phenoxyethylene glycol (meth)acrylate, phenoxybenzyl (meth)acrylate, stearyl (meth)acrylate, dicyclopentanyl (meth)acrylate, butoxydiethylene glycol (meth)acrylate, and methoxypolyethylene glycol (meth)acrylate. Preferred among these are benzyl methacrylate, isobornyl methacrylate, and p-cumyl-phenoxyethylene glycol methacrylate.

Examples of the hydrophobic aromatic bifunctional polymerizable monomers include 2,2-bis((meth)acryloyloxyphenyl)propane, 2,2-bis[4-(2-hydroxy-3-(meth)acryloyloxypropoxy)phenyl]propane, 2,2-bis(4-(meth)acryloyloxyethoxyphenyl)propane, 2,2-bis(4-(meth)acryloyloxypolyethoxyphenyl)propane, 2,2-bis(4-(meth)acryloyloxydiethoxyphenyl)propane, 2,2-bis(4-(meth)acryloyloxytriethoxyphenyl)propane, 2,2-bis(4-(meth)acryloyloxytetraethoxyphenyl)propane, 2,2-bis(4-(meth)acryloyloxypentaethoxyphenyl)propane, 2,2-bis(4-(meth)acryloyloxydipropoxyphenyl)propane, 2-(4-(meth)acryloyloxydiethoxyphenyl)-2-(4-(meth)acryloyloxyethoxyphenyl)propane, 2-(4-(meth)acryloyloxydiethoxyphenyl)-2-(4-(meth)acryloyloxytriethoxyphenyl)propane, 2-(4-(meth)acryloyloxydipropoxyphenyl)-2-(4-(meth)acryloyloxytriethoxyphenyl)propane, 2,2-bis(4-(meth)acryloyloxypropoxyphenyl)propane, and 2,2-bis(4-(meth)acryloyloxyisopropoxyphenyl)propane. Preferred among these are 2,2-bis[4-(2-hydroxy-3-methacryloyloxypropoxy)phenyl]propane (commonly known as Bis-GMA), 2,2-bis(4-methacryloyloxyethoxyphenyl)propane, 2,2-bis(4-methacryloyloxypolyethoxyphenyl)propane (with an average of 2.6 moles of ethoxy group added; commonly known as D-2.6E), 2,2-bis(4-methacryloyloxydiethoxyphenyl)propane, 2,2-bis(4-methacryloyloxytriethoxyphenyl)propane, 2,2-bis(4-methacryloyloxytetraethoxyphenyl)propane, and 2,2-bis(4-methacryloyloxypentaethoxyphenyl)propane. More preferred are Bis-GMA and D-2.6E.

Examples of the aliphatic bifunctional polymerizable monomers include monoethylene glycol di(meth)acrylate, diethylene glycol di(meth)acrylate, propylene glycol di(meth)acrylate, butylene glycol di(meth)acrylate, neopentyl glycol di(meth)acrylate, 1,5-pentanediol di(meth)acrylate, 1,6-hexanediol di(meth)acrylate, 1,10-decanediol di(meth)acrylate, and 2,2,4-trimethylhexamethylenebis(2-carbamoyloxyethyl)di(meth)acrylate. Preferred among these are neopentyl glycol dimethacrylate (commonly known as NPG), 2,2,4-trimethylhexamethylenebis(2-carbamoyloxyethyl)dimethacrylate (commonly known as UDMA), 1,10-decanediol dimethacrylate (commonly known as DD), and 2,2,4-trimethylhexamethylenebis(2-carbamoyloxyethyl)dimethacrylate. More preferred are NPG, UDMA, and DD.

Examples of the tri- and higher-functional polymerizable monomers include trimethylolpropane tri(meth)acrylate, trimethylolethane tri(meth)acrylate, trimethylolmethane tri(meth)acrylate, pentaerythritol tri(meth)acrylate, pentaerythritol tetra(meth)acrylate, dipentaerythritol penta(meth)acrylate, N,N-(2,2,4-trimethylhexamethylene)bis[2-(aminocarboxy)propane-1,3-diol]tetra(meth)acrylate, and 1,7-diacryloyloxy-2,2,6,6-tetra(meth)acryloyloxymethyl-4-oxaheptane. Preferred among these is N,N-(2,2,4-trimethylhexamethylene)bis[2-(aminocarboxy)propane-1,3-diol]tetramethacrylate.

In view of the paste flowability and the mechanical strength of the cured product, preferred among these hydrophobic polymerizable monomers (A-1b) are aromatic bifunctional polymerizable monomers, and aliphatic bifunctional polymerizable monomers.

The hydrophobic polymerizable monomer (A-1b) may be incorporated alone, or two or more thereof may be used in combination.

An excessively high content of hydrophobic polymerizable monomer (A-1b) in a curable dental composition of the present invention may lead to a reduction in the wettability of the curable dental composition to tooth structure, and a subsequent decrease in adhesion. When the content is too low, the strong hydrophilic interaction between the polymerizable monomer and the filler may result in inadequate paste flowability, possibly leading to a cured product with insufficient mechanical strength.

The content of the hydrophobic polymerizable monomer (A-1b) in a curable dental composition of the present invention is therefore preferably 55 parts or more by mass, more preferably 70 parts or more by mass, even more preferably 85 parts or more by mass, particularly preferably 90 parts or more by mass, and may be 100 parts by mass in total 100 parts by mass of polymerizable monomer (A).

### <Polymerizable Monomer Having Acidic Group (A-2)>

A curable dental composition of the present invention may comprise a polymerizable monomer having an acidic group (A-2). The polymerizable monomer having an acidic group (A-2) used in the present invention is preferably a radical polymerizable monomer having at least one acidic group and at least one polymerizable group. In view of adhesive properties, the acidic group is preferably an acidic group such as a phosphoric acid group, a pyrophosphoric acid group, a thiophosphoric acid group, a phosphonic acid group, a carboxylic acid group, or a sulfonic acid group. In view of ease of radical polymerization, the polymerizable group is preferably a (meth)acryl group and/or a (meth)acrylamide group.

Examples of polymerizable monomers having a phosphoric acid group include 2-(meth)acryloyloxyethyl dihydrogen phosphate, 3-(meth)acryloyloxypropyl dihydrogen phosphate, 4-(meth)acryloyloxybutyl dihydrogen phosphate, 5-(meth)acryloyloxypentyl dihydrogen phosphate, 6-(meth)acryloyloxyhexyl dihydrogen phosphate, 7-(meth)acryloyloxyheptyl dihydrogen phosphate, 8-(meth)acryloyloxyoctyl dihydrogen phosphate, 9-(meth)acryloyloxynonyl dihydrogen phosphate, 10-(meth)acryloyloxydecyl dihydrogen phosphate, 11-(meth)acryloyloxyundecyl dihydrogen phosphate, 12-(meth)acryloyloxydodecyl dihydrogen phosphate, 16-(meth)acryloyloxyhexadecyl dihydrogen phosphate, 20-(meth)acryloyloxyicosyl dihydrogen phosphate, bis[2-(meth)acryloyloxyethyl]hydrogen phosphate, bis[4-(meth)acryloyloxybutyl]hydrogen phosphate, bis[6-(meth)acryloyloxyhexyl]hydrogen phosphate, bis[8-(meth)acryloyloxyoctyl]hydrogen phosphate, bis[9-(meth)acryloyloxynonyl]hydrogen phosphate, bis[10-(meth)acryloyloxydecyl]hydrogen phosphate, 1,3-di(meth)acryloyloxypropyl dihydrogen phosphate, 2-(meth)acryloyloxyethylphenyl hydrogen phosphate, 2-(meth)acryloyloxyethyl-(2-bromoethyl)hydrogen phosphate, 2-(meth)acryloyloxyethyl-(4-methoxyphenyl)hydrogen phosphate, 2-(meth)acryloyloxypropyl-(4-methoxyphenyl)hydrogen phosphate, and acid chlorides, alkali metal salts, and amine salts of these.

Examples of polymerizable monomers having a pyrophosphoric acid group include bis[2-(meth)acryloyloxyethyl]pyrophosphate, bis[4-(meth)acryloyloxybutyl]pyrophosphate, bis[6-(meth)acryloyloxyhexyl]pyrophosphate, bis[8-(meth)acryloyloxyoctyl]pyrophosphate, bis[10-(meth)acryloyloxydecyl]pyrophosphate, and acid chlorides, alkali metal salts, and amine salts of these.

Examples of polymerizable monomers having a thiophosphoric acid group include 2-(meth)acryloyloxyethyl dihydrogen thiophosphate, 3-(meth)acryloyloxypropyl dihydrogen thiophosphate, 4-(meth)acryloyloxybutyl dihydrogen thiophosphate, 5-(meth)acryloyloxypentyl dihydrogen thiophosphate, 6-(meth)acryloyloxyhexyl dihydrogen thiophosphate, 7-(meth)acryloyloxyheptyl dihydrogen thiophosphate, 8-(meth)acryloyloxyoctyl dihydrogen thiophosphate, 9-(meth)acryloyloxynonyl dihydrogen thiophosphate, 10-(meth)acryloyloxydecyl dihydrogen thiophosphate, 11-(meth)acryloyloxyundecyl dihydrogen thiophosphate, 12-(meth)acryloyloxydodecyl dihydrogen thiophosphate, 16-(meth)acryloyloxyhexadecyl dihydrogen thiophosphate, 20-(meth)acryloyloxyicosyl dihydrogen thiophosphate, and acid chlorides, alkali metal salts, and ammonium salts of these.

Examples of polymerizable monomers having a phosphonic acid group include 2-(meth)acryloyloxyethylphenyl phosphonate, 5-(meth)acryloyloxypentyl-3-phosphonopropionate, 6-(meth)acryloyloxyhexyl-3-phosphonopropionate, 10-(meth)acryloyloxydecyl-3-phosphonopropionate, 6-(meth)acryloyloxyhexylphosphonoacetate, 10-(meth)acryloyloxydecylphosphonoacetate, and acid chlorides, alkali metal salts, and ammonium salts of these.

Examples of polymerizable monomers having a carboxylic acid group include monofunctional (meth)acrylic acid esters having one carboxyl group or an acid anhydride group thereof within the molecule, monofunctional (meth)acrylic acid esters having multiple carboxyl groups or acid anhydride groups thereof within the molecule, and acid chlorides, alkali metal salts, and ammonium salts of these.

Examples of the monofunctional polymerizable monomers having one carboxyl group or an acid anhydride group thereof within the molecule include (meth)acrylic acid, N-(meth)acryloyl glycine, N-(meth)acryloyl aspartic acid, 2-(meth)acryloyloxyethyl hydrogen succinate, 2-(meth)acryloyloxyethyl hydrogen phthalate, 2-(meth)acryloyloxyethyl hydrogen malate, O-(meth)acryloyl tyrosine, N-(meth)acryloyl tyrosine, N-(meth)acryloyl phenylalanine, N-(meth)acryloyl-p-aminobenzoic acid, N-(meth)acryloyl-o-aminobenzoic acid, 2-(meth)acryloyloxybenzoic acid, 3-(meth)acryloyloxybenzoic acid, 4-(meth)acryloyloxybenzoic acid, N-(meth)acryloyl-5-aminosalicylic acid, N-(meth)acryloyl-4-aminosalicylic acid, and compounds derived by converting the carboxyl group of the aforementioned compounds into an acid anhydride group

Examples of the monofunctional polymerizable monomers having multiple carboxyl groups or acid anhydride groups thereof within the molecule include 6-(meth)acryloyloxyhexane-1,1-dicarboxylic acid, 9-(meth)acryloyloxynonane-1,1-dicarboxylic acid, 10-(meth)acryloyloxydecane-1,1-dicarboxylic acid, 11-(meth)acryloyloxyundecane-1,1-dicarboxylic acid, 12-(meth)acryloyloxydodecane-1,1-dicarboxylic acid, 13-(meth)acryloyloxytridecane-1,1-dicarboxylic acid, 4-(meth)acryloyloxyethyl trimellitate, 4-(meth)acryloyloxyethyl trimellitate anhydride, 4-(meth)acryloyloxybutyl trimellitate, 4-(meth)acryloyloxyhexyl trimellitate, 4-(meth)acryloyloxydecyl trimellitate, 2-(meth)acryloyloxyethyl-3'-(meth)acryloyloxy-2'-(3,4-dicarboxybenzoyloxy)propyl succinate, 6-(meth)acryloyloxyethylnaphthalene-1,2,6-tricarboxylic anhydride, 6-(meth)acryloyloxyethylnaphthalene-2,3,6-tricarboxylic anhydride, 4-(meth)acryloyloxyethylcarbonylpropionoyl-1,8-naphthalic anhydride, and 4-(meth)acryloyloxyethylnaphthalene-1,8-tricarboxylic anhydride.

Examples of polymerizable monomers having a sulfonic acid group include 2-sulfoethyl (meth)acrylate, and acid chlorides, alkali metal salts, and ammonium salts thereof.

In view of providing favorable bond strength as a curable dental composition, preferred among these polymerizable monomers having an acidic group (A-2) are polymerizable monomers having a phosphoric acid group, or polymerizable monomers having a carboxylic acid group, more preferably 2-(meth)acryloyloxyethyl dihydrogen phosphate, 3-(meth)acryloyloxypropyl dihydrogen phosphate, 4-(meth)acryloyloxybutyl dihydrogen phosphate, 5-(meth)acryloyloxypentyl dihydrogen phosphate, 6-(meth)acryloyloxyhexyl dihydrogen phosphate, 7-(meth)acryloyloxyheptyl dihydrogen phosphate, 8-(meth)acryloyloxyoctyl dihydrogen phosphate, 9-(meth)acryloyloxynonyl dihydrogen phosphate, 10-(meth)acryloyloxydecyl dihydrogen phosphate, 11-(meth)acryloyloxyundecyl dihydrogen phosphate, 12-(meth)acryloyloxydodecyl dihydrogen phosphate, 16-(meth)acryloyloxyhexadecyl dihydrogen phosphate, 20-(meth)acryloyloxyicosyl dihydrogen phosphate, 4-(meth)acryloyloxyethyl trimellitate anhydride, 4-(meth)acryloyloxyethyl trimellitate, 11-(meth)acryloyloxyundecane-1,1-dicarboxylic acid, and a mixture of 2-(meth)acryloyloxyethyl dihydrogen phosphate and bis(2-methacryloyloxyethyl)hydrogen phosphate, even more preferably 8-(meth)acryloyloxyoctyl dihydrogen phosphate, 9-(meth)acryloyloxynonyl dihydrogen phosphate, 10-(meth)acryloyloxydecyl dihydrogen phosphate, 11-(meth)acryloyloxyundecyl dihydrogen phosphate, 12-(meth)acryloyloxydodecyl dihydrogen phosphate, 16-(meth)acryloyloxyhexadecyl dihydrogen phosphate, and 20-(meth)acryloyloxyicosyl dihydrogen phosphate. In view of a balance with curability, 10-(meth)acryloyloxydecyl dihydrogen phosphate is most preferred.

The polymerizable monomer having an acidic group (A-2) may be incorporated alone, or two or more thereof may be used in combination.

In view of adhesive properties to tooth structure, the content of the polymerizable monomer having an acidic group (A-2) in a curable dental composition of the present invention is preferably 0 to 30 parts by mass, more preferably 0 to 20 parts by mass, even more preferably 0 to 15 parts by mass in total 100 parts by mass of polymerizable monomer (A).

### [Filler (B)]

It is required that a curable dental composition of the present invention comprise a filler (B) having an average particle diameter of 1 µm or more, in order to increase the mechanical strength of the cured product.

Examples of filler (B) include inorganic fillers, organic fillers, and organic-inorganic composite fillers.

Examples of inorganic filler components include the following:
various types of glass (containing silica as a main component, and, optionally, oxides of elements such as heavy metals, boron, and aluminum), including, for example, glass powders of common compositions, such as fused silica, quartz, soda-lime-silica glass, E glass, C glass, and borosilicate glass (PYREX^{®} glass), and dental glass powders such as barium glass (GM27884 and 8235 manufactured by SCHOTT, and E-2000 and E-3000 manufactured by ESSTECH), strontium·borosilicate glass (E-4000 manufactured by ESSTECH), lanthanum glass-ceramic (GM31684 manufactured by SCHOTT), and fluoroaluminosilicate glass (GM35429, G018-091, and G018-117 manufactured by SCHOTT); and
various types of ceramics, composite oxides (such as silica-titania, and silica-zirconia), diatomaceous earth, kaolin, clay minerals (such as montmorillonite), activated earth, synthetic zeolite, mica, calcium fluoride, ytterbium fluoride, yttrium fluoride, core-shell structured calcium fluoride with silica coating, core-shell structured ytterbium fluoride with silica coating, core-shell structured yttrium fluoride with silica coating, calcium phosphate, barium sulfate, zirconium dioxide, titanium dioxide, hydroxyapatite, core-shell structured calcium phosphate with silica coating, core-shell structured barium sulfate with silica coating, core-shell structured zirconium dioxide with silica coating, core-shell structured titanium dioxide with silica coating, and core-shell structured hydroxyapatite with silica coating.

In view of strength and other properties, preferred among these are various types of glass, composite oxides (such as silica-titania, and silica-zirconia), core-shell structured calcium fluoride with silica coating, core-shell structured ytterbium fluoride with silica coating, core-shell structured yttrium fluoride with silica coating, core-shell structured calcium phosphate with silica coating, core-shell structured barium sulfate with silica coating, core-shell structured zirconium dioxide with silica coating, core-shell structured titanium dioxide with silica coating, and core-shell structured hydroxyapatite with silica coating.

Examples of organic filler components include polymethyl methacrylate, polyethyl methacrylate, a methyl methacrylate-ethyl methacrylate copolymer, crosslinked polymethyl methacrylate, crosslinked polyethyl methacrylate, polyamide, polyvinyl chloride, polystyrene, chloroprene rubber, nitrile rubber, an ethylene-vinyl acetate copolymer, a styrene-butadiene copolymer, an acrylonitrile-styrene copolymer, and an acrylonitrile-styrene-butadiene copolymer.

The organic-inorganic composite filler is a filler obtained by adding a polymerizable monomer to the inorganic filler, polymerizing the mixture in paste form, and pulverizing the resulting material. The organic-inorganic composite filler may be, for example, a TMPT filler (a filler obtained by mixing trimethylolpropane methacrylate and silica filler, and pulverizing the mixture after polymerization).

The filler (B) may be spherical or irregular in shape, for example.

In a curable dental composition of the present invention, in view of facilitating both paste flowability under applied pressure and shape retention at rest through cooperative action with rheology modifier (E), preferred for use as filler (B) is an irregularly shaped filler (B-2), more preferably a combination of spherical filler (B-1) and irregularly shaped filler (B-2).

### <Spherical Filler (B-1)>

The spherical filler (B-1) is a filler representing particles that appear round in shape when observed in a unit field of an electron micrograph, and that have an average uniformity of 0.6 or more as calculated by dividing the diameter of a particle perpendicular to its maximum diameter by the maximum diameter.

In view of even superior sliding effect, the spherical filler (B-1) in a curable dental composition of the present invention has an average uniformity of preferably 0.7 or more, more preferably 0.8 or more.

The material of spherical filler (B-1) is not particularly limited because the spherical filler (B-1) can produce a sliding effect by being spherical, and mitigate interference between fillers.

A certain embodiment is, for example, a curable dental composition in which the spherical filler (B-1) is an inorganic filler.

The spherical filler (B-1) used in the present invention does not necessarily need to be produced or acquired as a singular particle, and may be one obtained by mixing two or more fillers of different average particle diameters or different components, provided that the average particle diameter of each filler falls within the foregoing ranges.

### <Irregularly Shaped Filler (B-2)>

The irregularly shaped filler (B-2) used in the present invention is a filler that does not appear round in shape when observed in a unit field of an electron micrograph, and that has an average uniformity of less than 0.6 as calculated by dividing the diameter of a particle perpendicular to its maximum diameter by the maximum diameter.

The irregularly shaped filler (B-2) does not necessarily need to be produced or acquired as a singular particle, and may be one obtained by mixing two or more fillers of different average particle diameters or different components, provided that the average particle diameter of each filler falls within the foregoing ranges.

The filler (B) used in the present invention has an average particle diameter of 1.0 to 6.0 µm. In view of mechanical strength and coating thickness, the average particle diameter of filler (B) is more preferably 1.0 to 5.0 µm, even more preferably 1.0 to 3.0 µm. When the average particle diameter is more than 6.0 µm, the dispersibility of the irregularly shaped filler in the curable dental composition deteriorates, and a uniform paste cannot be prepared.

In view of paste flowability, shape retention at rest, and the mechanical strength of the cured product, the content of the filler (B) in a curable dental composition of the present invention is preferably 50 to 2,000 parts by mass with respect to total 100 parts by mass of polymerizable monomer (A). In view of providing even superior effects, the content of filler (B) is more preferably 100 to 1,500 parts by mass, even more preferably 150 to 1,000 parts by mass with respect to total 100 parts by mass of polymerizable monomer (A).

In certain embodiments, in view of superior shape retention at rest and superior mechanical strength of the cured product, the content of the filler (B) in a curable dental composition of the present invention is preferably 45 to 95 mass%, more preferably 55 to 92 mass%, even more preferably 60 to 90 mass% in total 100 mass% of the composition.

In this specification, the average particle diameters of filler (B) and inorganic fine particle (E-1) can be determined using a laser diffraction scattering method. The inorganic fine particle (E-1) can also be measured by electron microscopy, as will be described later.

As a specific example, the average particle diameters of filler (B) and inorganic fine particle (E-1) may be measured by volume using, for example, a laser diffraction particle size analyzer (SALD-2300 manufactured by Shimadzu Corporation) with a 0.2% sodium hexametaphosphate aqueous solution used as dispersion medium.

### [Hydrophobizing Agent (x-1)]

Preferably, the filler (B) in the present invention is one that is surface-treated with a hydrophobizing agent (x-1) to improve its dispersibility in the polymerizable monomer. The hydrophilic interaction with the polymerizable monomer can be diminished as long as the filler is hydrophobized. Accordingly, the hydrophobizing agent (x-1) is not particularly limited, and any known surface treatment agent can be used so long as it is capable of imparting hydrophobicity.

Typical examples of hydrophobizing agent (x-1) include silane coupling agents, titanate-based coupling agents, aluminate-based coupling agents, and zirco-aluminate-based coupling agents. In view of enhancing the effectiveness of the present invention, particularly preferred is a treatment with a silane coupling agent.

The hydrophobizing agent (x-1) may be used alone, or two or more thereof may be used in combination. In the case where two or more hydrophobizing agents (x-1) are used in combination, the resultant surface treatment layer may be a mixture of two or more surface treatment agents, or may be a multilayer structure with a plurality of surface treatment layers. The surface treatment method is not particularly limited, and known methods can be used.

The silane coupling agent is not particularly limited, and known silane coupling agents may be used, as long as it can impart hydrophobicity. However, in view of enhancing the effectiveness of the present invention, preferred for use is a silane coupling agent having a polymerizable group and represented by the general formula (1) below. By using a silane coupling agent having a polymerizable group and represented by the following general formula (1), the filler dispersibility improves, leading to enhanced paste flowability. Additionally, the mechanical strength of the cured product improves through polymerization of the surface treatment layer of the filler and each type of polymerizable monomer.

Y-SiRₙX₍₃₋ₙ₎ (1),

wherein Y represents a polymerizable group, or a monovalent organic group having a polymerizable group, R represents a group selected from the group consisting of an alkyl group, an aryl group, and an aralkyl group, X represents a hydroxyl group or a hydrolyzable group, n represents an integer of 0, 1, or 2, and R may be the same or different from each other when R is plural, and X may be the same or different from each other when X is plural.

The polymerizable group present in Y is not particularly limited, and may be, for example, a (meth)acryloyl group, a vinyl group, a mercapto group, a (meth)allyl group, or an epoxy group. In view of mechanical strength and other properties, preferred are (meth)acryloyl groups, more preferably methacryloyl groups.

When Y is a monovalent organic group having a polymerizable group, and the polymerizable group is bound to the organic group, the polymerizable group may be bound to the organic group either directly or via a divalent group containing a heteroatom such as an oxygen atom or nitrogen atom.

For example, Y may be a group resulting from the bonding of a (meth)acryloyl group and an organic group via a divalent group containing an oxygen atom.

As another example, when the polymerizable group present in Y is a (meth)acryloyl group, part of Y may form a (meth)acryloyloxy group or a (meth)acrylamide group

The number of polymerizable groups present in Y is not particularly limited, and is preferably 1 to 4, more preferably 1 or 2, even more preferably 1. When Y has a plurality of polymerizable groups, the polymerizable groups may be the same or different from each other.

Y may represent only polymerizable groups such as above, or may be a monovalent organic group having a polymerizable group. When Y is a monovalent organic group having a polymerizable group, Y may be a group in which the functional group and the organic group are bound to each other either directly or indirectly via a divalent group having a heteroatom such as an oxygen atom or nitrogen atom. The organic group is not particularly limited, and may be, for example, an alkyl group having 1 to 20 carbon atoms, an aryl group having 6 to 18 carbon atoms, or an aralkyl group having 7 to 26 carbon atoms. For considerations such as further improvement of adhesive properties to both dental restoration materials and tooth structures, preferred among these are alkyl groups having 1 to 20 carbon atoms, more preferably alkyl groups having 3 to 11 carbon atoms, even more preferably alkyl groups having 5 to 11 carbon atoms. Examples of alkyl groups having 5 to 11 carbon atoms include n-pentyl groups, n-hexyl groups, n-octyl groups, and n-undecyl groups. Preferred are n-octyl groups and n-undecyl groups.

Examples of Y include (meth)acryloyloxyalkyl groups (such as (meth)acryloyloxymethyl groups, (meth)acryloyloxyethyl groups, and 3-(meth)acryloyloxypropyl groups), γ-(meth)acrylamidepropyl groups, vinyl groups, (meth)allyl groups, and γ-glycidoxypropyl groups. Preferred are (meth)acryloyloxymethyl groups, (meth)acryloyloxyethyl groups, 3-(meth)acryloyloxypropyl groups, γ-(meth)acryloyloxyoctyl groups, and γ-(meth)acryloyloxyundecyl groups. More preferred are γ-(meth)acryloyloxyoctyl groups, and γ-(meth)acryloyloxyundecyl groups.

The alkyl group represented by R is not particularly limited, and may be, for example, an alkyl group having 1 to 5 carbon atoms. Specific examples include methyl groups, ethyl groups, n-propyl groups, isopropyl groups, n-butyl groups, and n-pentyl groups.

The aryl group represented by R is not particularly limited, and may be, for example, an aryl group having 6 to 10 carbon atoms. Specific examples include phenyl groups and naphthyl groups.

The aralkyl group represented by R is not particularly limited, and may be, for example, an aralkyl group having 7 to 12 carbon atoms. Specific examples include benzyl groups.

In view of mechanical strength and other properties, R is preferably an alkyl group, more preferably an alkyl group having 1 to 5 carbon atoms, even more preferably a methyl group.

The hydrolyzable group represented by X may be a group that, through hydrolysis, has the capability to form a silanol group with the silicon atom to which it is attached. Examples include alkoxy groups, acyloxy groups, siloxy groups, and halogen atoms.

The alkoxy group is not particularly limited, and may be, for example, an alkoxy group having 1 to 5 carbon atoms. Specific examples include methoxy groups, ethoxy groups, n-propoxy groups, isopropoxy groups, n-butoxy groups, and n-pentyloxy groups.

The acyloxy group is not particularly limited, and may be, for example, an acyloxy group having 1 to 5 carbon atoms. Specific examples include formyloxy groups, acetoxy groups, n-propionyloxy groups, isopropionyloxy groups, n-butanoyloxy groups, and n-pentanoyloxy groups.

The siloxy group is not particularly limited, and may be, for example, a trimethylsiloxy group.

The halogen atom is not particularly limited, and may be, for example, a chlorine atom or a bromine atom.

Preferably, X is an alkoxy group, more preferably an alkoxy group having 1 to 5 carbon atoms, even more preferably a methoxy group or an ethoxy group.

The symbol n represents an integer of 0 to 2. In view of properties such as the mechanical strength of the cured product of the curable dental composition, n is preferably 0 or 1. When n is 0 or 1, the multiple X may be the same or different from each other. When n is 2, the multiple R may be the same or different from each other.

Examples of the silane coupling agent include silane coupling agents having a polymerizable group and represented by general formula (1), such as:
silane compounds containing a (meth)acryloyl group, such as (meth)acryloyloxymethyltrimethoxysilane, 2-(meth)acryloyloxyethyltrimethoxysilane, 3-(meth)acryloyloxypropyltrimethoxysilane, 4-(meth)acryloyloxybutyltrimethoxysilane, 5-(meth)acryloyloxypentyltrimethoxysilane, 6-(meth)acryloyloxyhexyltrimethoxysilane, 7-(meth)acryloyloxyheptyltrimethoxysilane, 8-(meth)acryloyloxyoctyltrimethoxysilane, 9-(meth)acryloyloxynonyltrimethoxysilane, 10-(meth)acryloyloxydecyltrimethoxysilane, 11-(meth)acryloyloxyundecyltrimethoxysilane, 11-(meth)acryloyloxyundecyldichloromethylsilane, 11-(meth)acryloyloxyundecyltrichlorosilane, 11-(meth)acryloyloxyundecyldimethoxymethylsilane, 12-(meth)acryloyloxydodecyltrimethoxysilane, 13-(meth)acryloyloxytridecyltrimethoxysilane, 3-(meth)acryloyloxypropylmethyldimethoxysilane, 3-(meth)acryloyloxypropylmethyldiethoxysilane, 3-(meth)acryloyloxypropylmethyldiisopropoxysilane, 3-(meth)acryloyloxypropylmethyldi(trimethylsiloxy)silane, 3-(meth)acryloyloxypropylmethyldi(hexylsiloxy)silane, 3-(meth)acryloyloxypropyltris(trimethylsiloxy)silane, (meth)acryloyloxy-2-(2-vinyloxyethoxy)ethylmethyldimethoxysilane, 6-(meth)acryloyloxyhexylmethyldimethoxysilane, (meth)acryloyloxy-p-phenylethylmethyldimethoxysilane, 6-(meth)acryloyloxyhexylmethyldiethoxysilane, 10-(meth)acryloyloxydecylmethyldimethoxysilane, 11-(meth)acryloyloxyundecylmethyldimethoxysilane, 11-(meth)acryloyloxyundecylmethyldiethoxysilane, 11-(meth)acryloyloxyundecylmethyldihexyloxysilane, 20-(meth)acryloyloxyeicosylmethyldimethoxysilane, 3-(meth)acryloyloxypropylphenyldimethoxysilane, 3-(meth)acryloyloxypropylmethyldichlorosilane, 11-(meth)acryloyloxyundecylmethyldichlorosilane, 11-(meth)acryloyloxyundecylethyldichlorosilane, 3-(meth)acryloyloxypropyldimethylmonomethoxysilane, 3-(meth)acryloyloxypropyldimethylmonoethoxysilane, 3-(meth)acryloyloxypropyldimethylmonoisopropoxysilane, 3-(meth)acryloyloxypropyldimethylmonotrimethylsiloxysilane, 3-(meth)acryloyloxypropyldimethylmonohexyloxysilane, (meth)acryloyloxy-2-(2-vinyloxyethoxy)ethyldimethylmonomethoxysilane, 6-(meth)acryloyloxyhexyldimethylmonomethoxysilane, (meth)acryloyloxy-p-phenylethyldimethylmonomethoxysilane, 6-(meth)acryloyloxyhexyldimethylmonoethoxysilane, 10-(meth)acryloyloxydecyldimethylmonomethoxysilane, 11-(meth)acryloyloxyundecyldimethylmonomethoxysilane, 11-(meth)acryloyloxyundecyldimethylmonoethoxysilane, 11-(meth)acryloyloxyundecyldimethylmonohexyloxysilane, 20-(meth)acryloyloxyeicosyldimethylmonomethoxysilane, 3-(meth)acryloyloxypropyldiphenylmonomethoxysilane, 3-(meth)acryloyloxypropyldimethylmonochlorosilane, 11-(meth)acryloyloxyundecyldimethylmonochlorosilane, and 11-(meth)acryloyloxyundecyldiethylmonochlorosilane;
silane compounds containing a vinyl group, such as vinylmethyldimethoxysilane, vinylmethyldiethoxysilane, vinylmethyldichlorosilane, vinylmethyldiacetoxysilane, vinylmethyldi(2-methoxyethoxy)silane, vinyldimethylmonomethoxysilane, vinyldimethylmonoethoxysilane, vinyldimethylmonochlorosilane, vinyldimethylmonoacetoxysilane, and vinyldimethylmono(2-methoxyethoxy)silane;
silane compounds containing an epoxy group, such as 3-glycidoxypropylmethyldimethoxysilane, 3-glycidoxypropylmethyldiethoxysilane, 3-glycidoxypropyldimethylmonomethoxysilane, and 3-glycidoxypropyldimethylmonoethoxysilane; and
silane compounds containing a (meth)allyl group, such as allylmethyldiethoxysilane, and allyldimethylmonoethoxysilane.

Other examples of the silane coupling agent include silane coupling agents having no polymerizable group, such as hexaethyldisilazane, hexa n-propyldisilazane, hexaisopropyldisilazane, 1,1,2,2-tetramethyl-3,3-diethyldisilazane, 1,1,3,3-tetramethyldisilazane, 1,1,1,3,3,3-hexamethyldisilazane, and 1,1,1,3,3-pentamethyldisilazane.

The silane coupling agent may be a hydrolysate and/or condensate of these. The silane coupling agent may be used alone, or two or more thereof may be used in combination.

### [Polymerization Initiator (C)]

The polymerization initiator (C) is classified into photopolymerization initiator (C-1) and chemical polymerization initiator (C-2). In view of the ability to more swiftly remove the excess portion by preliminary irradiation, it is required that a curable dental composition of the present invention comprise a photopolymerization initiator (C-1) as polymerization initiator (C).

The polymerization initiator (C) may be solely a photopolymerization initiator (C-1), or may be a combination of photopolymerization initiator (C-1) and chemical polymerization initiator (C-2).

### <Photopolymerization Initiator (C-1)>

The photopolymerization initiator (C-1) is not particularly limited, and may be any known photopolymerization initiator with no limitation. Examples of known photopolymerization initiators include α-diketones, ketals, thioxanthones, (bis)acylphosphine oxides, and α-aminoacetophenones.

As another example of photopolymerization initiator (C-1), it is also possible to use the triazine compounds substituted with a trihalomethyl group(s) described in JP 2014-111555 A.

Examples of the α-diketones include camphorquinone (commonly known as CQ), benzil, and 2,3-pentanedione.

Examples of the ketals include benzyl dimethyl ketal, and benzyl diethyl ketal.

Examples of the thioxanthones include 2-chlorothioxanthone, and 2,4-diethylthioxanthone.

Examples of the (bis)acylphosphine oxides include 2,4,6-trimethylbenzoyldiphenylphosphine oxide, bis(2,4,6-trimethylbenzoyl)phenylphosphine oxide, dibenzoylphenylphosphine oxide, bis(2,6-dimethoxybenzoyl)phenylphosphine oxide, tris(2,4-dimethylbenzoyl)phosphine oxide, tris(2-methoxybenzoyl)phosphine oxide, 2,6-dimethoxybenzoyldiphenylphosphine oxide, 2,6-dichlorobenzoyldiphenylphosphine oxide, 2,3,5,6-tetramethylbenzoyldiphenylphosphine oxide, benzoyl-bis(2,6-dimethylphenyl)phosphine oxide, 2,4,6-trimethylbenzoylethoxyphenylphosphine oxide, the water-soluble acylphosphine oxide compounds disclosed in JP H03-57916 B, and salts of these (for example, sodium salts of 2,4,6-trimethylbenzoylphenylphosphine oxide).

Examples of the α-aminoacetophenones include 2-benzyl-2-dimethylamino-1-(4-morpholinophenyl)-1-butanone, 2-benzyl-2-diethylamino-1-(4-morpholinophenyl)-1-butanone, 2-benzyl-2-dimethylamino-1-(4-morpholinophenyl)-1-propanone, 2-benzyl-2-diethylamino-1-(4-morpholinophenyl)-1-propanone, 2-benzyl-2-dimethylamino-1-(4-morpholinophenyl)-1-pentanone, and 2-benzyl-2-diethylamino-1-(4-morpholinophenyl)-1-pentanone.

In view of properties such as curability, the photopolymerization initiator (C-1) is preferably an α-diketone or an acylphosphine oxide, more preferably an α-diketone, even more preferably a camphorquinone.

The photopolymerization initiator (C-1) may be used alone, or two or more thereof may be used in combination.

In view of curability and storage stability, the content of photopolymerization initiator (C-1) is preferably 0.01 to 10 parts by mass, more preferably 0.02 to 5 parts by mass, even more preferably 0.03 to 3 parts by mass with respect to total 100 parts by mass of polymerizable monomer (A).

### <Chemical Polymerization initiator (C-2)>

The chemical polymerization initiator (C-2) is not particularly limited, and may be any known chemical polymerization initiator with no limitation. With a curable dental composition of the present invention comprising a chemical polymerization initiator (C-2), the resultant cured product can achieve even greater mechanical strength. Preferably, the chemical polymerization initiator (C-2) comprises organic peroxides and/or inorganic peroxides.

Examples of the organic peroxides include hydroperoxides, peroxyesters, ketone peroxides, peroxyketals, dialkyl peroxides, diacyl peroxides, and peroxydicarbonates. Preferred among these are hydroperoxides and peroxyesters.

Examples of the hydroperoxides include cumene hydroperoxide, t-butyl hydroperoxide, t-hexyl hydroperoxide, p-menthane hydroperoxide, diisopropylbenzene hydroperoxide, and 1,1,3,3-tetramethylbutyl hydroperoxide.

The peroxyesters may be any known peroxyesters with no limitation, provided that the peroxy group has an acyl group at one end, and a hydrocarbon group or a similar group at the other end. Specific examples include α,α-bis(neodecanoylperoxy)diisopropylbenzene, cumylperoxyneodecanoate, 1,1,3,3-tetramethylbutylperoxyneodecanoate, 1-cyclohexyl-1-methylethylperoxyneodecanoate, t-hexylperoxyneodecanoate, t-butylperoxyneodecanoate, t-hexylperoxypivalate, t-butylperoxypivalate, 1,1,3,3-tetramethylbutylperoxy-2-ethylhexanoate, 2,5-dimethyl-2,5-bis(2-ethylhexanoylperoxy)hexane, 1-cyclohexyl-1-methylethyl peroxy-2-ethylhexanoate, t-hexylperoxy-2-ethylhexanoate, t-butylperoxy-2-ethylhexanoate, t-butylperoxyisobutyrate, t-hexylperoxyisopropyl monocarbonate, t-butylperoxymaleic acid, t-butylperoxy-3,5,5-trimethylhexanoate, t-butylperoxylaurate, 2,5-dimethyl-2,5-bis(m-toluoylperoxy)hexane, t-butylperoxyisopropyl monocarbonate, t-butylperoxy-2-ethylhexyl monocarbonate, t-hexylperoxybenzoate, 2,5-dimethyl-2,5-bis(benzoylperoxy)hexane, t-butylperoxyacetate, t-butylperoxy-m-toluoylbenzoate, t-butylperoxybenzoate, and bis(t-butylperoxy)isophthalate. In view of storage stability and reactivity, preferred among these are t-butylperoxymaleic acid, t-butylperoxy-3,5,5-trimethylhexanoate, t-butylperoxybenzoate, t-butylperoxyisopropyl monocarbonate, t-butylperoxy-2-ethylhexyl monocarbonate, and t-butylperoxyacetate, more preferably t-butylperoxybenzoate.

Examples of the ketone peroxides include methyl ethyl ketone peroxide, cyclohexanone peroxide, methyl cyclohexanone peroxide, methyl acetoacetate peroxide, and acetyl acetone peroxide.

Examples of the peroxyketals include 1,1-bis(t-hexylperoxy)3,3,5-trimethylcyclohexane, 1,1-bis(t-hexylperoxy)cyclohexane, 1,1-bis(t-butylperoxy)3,3,5-trimethylcyclohexanone, 1,1-bis(t-butylperoxy)cyclohexane, 1,1-bis(t-butylperoxy)cyclodecane, 2,2-bis(t-butylperoxy)butane, n-butyl-4,4-bis(t-butylperoxy)valerate, and 2,2-bis(4,4-di-t-butylperoxycyclohexyl)propane.

Examples of the dialkyl peroxides include α,α-bis(t-butylperoxy)diisopropylbenzene, dicumyl peroxide, 2,5-dimethyl-2,5-bis(t-butylperoxy)hexane, t-butyl cumylperoxide, di-t-butyl peroxide, and 2,5-dimethyl-2,5-bis(t-butylperoxy)3-hexyne.

Examples of the diacyl peroxides include isobutyryl peroxide, 2,4-dichlorobenzoyl peroxide, 3,5,5-trimethylhexanoyl peroxide, octanoyl peroxide, lauroyl peroxide, stearyl peroxide, succinic acid peroxide, m-toluoylbenzoyl peroxide, and benzoyl peroxide.

Examples of the peroxydicarbonates include di-n-propylperoxy dicarbonate, diisopropylperoxy dicarbonate, bis(4-t-butylcyclohexyl)peroxydicarbonate, di(2-ethoxyethyl)peroxydicarbonate, di(2-ethylhexyl)peroxydicarbonate, di(2-methoxybutyl)peroxydicarbonate, and di(3-methyl-3-methoxybutyl)peroxydicarbonate.

Examples of the inorganic peroxides include peroxydisulfates and peroxydiphosphates. In view of reactivity, peroxydisulfates are preferred. Examples of the peroxydisulfates include sodium peroxydisulfate, potassium peroxydisulfate, aluminum peroxydisulfate, and ammonium peroxydisulfate. Preferred among these are sodium peroxydisulfate, potassium peroxydisulfate, and ammonium peroxydisulfate.

The chemical polymerization initiator (C-2) may be incorporated alone, or two or more thereof may be used in combination.

In view of bond strength to the adherend and working time margin, the content of chemical polymerization initiator (C-2) is preferably 0.001 to 20 parts by mass, more preferably 0.005 to 10 parts by mass, even more preferably 0.025 to 5 parts by mass with respect to total 100 parts by mass of polymerizable monomer (A). It is easier to provide adequate adhesive properties when the content of chemical polymerization initiator (C-2) is 0.001 parts or more by mass, whereas a content of 10 parts or less by mass facilitates maintaining the working time margin.

### [Polymerization Accelerator (D)]

A curable dental composition of the present invention may comprise a polymerization accelerator (D). The polymerization accelerator (D) is a compound that accelerates a reaction in conjunction with the polymerization initiator (C). The polymerization accelerator (D) is classified into photopolymerization accelerator (D-1) and chemical polymerization accelerator (D-2).

The polymerization accelerator (D) may comprise either one of photopolymerization accelerator (D-1) and chemical polymerization accelerator (D-2), or may comprise both photopolymerization accelerator (D-1) and chemical polymerization accelerator (D-2).

### <Photopolymerization Accelerator (D-1)>

The photopolymerization accelerator (D-1) is not particularly limited, and may be any known photopolymerization accelerator with no limitation.

Examples of the photopolymerization accelerator (D-1) used in a curable dental composition of the present invention include aldehydes, thiol compounds, aminobenzoic acid ester compounds, and amine-based reducing agents.

Examples of the aldehydes include terephthalaldehyde and benzaldehyde derivatives. Examples of the benzaldehyde derivatives include dimethylaminobenzaldehyde, p-methoxybenzaldehyde, p-ethoxybenzaldehyde, and p-n-octyloxybenzaldehyde.

Examples of the thiol compounds include 2-mercaptobenzoxazole, decanethiol, 3-mercaptopropyltrimethoxysilane, and thiobenzoic acid.

Examples of the aminobenzoic acid ester compounds include ethyl 4-(N,N-dimethylamino)benzoate, methyl 4-(N,N-dimethylamino)benzoate, n-butoxyethyl 4-N,N-(dimethylamino)benzoate, 2-[(meth)acryloyloxy]ethyl 4-(N,N-dimethylamino)benzoate, 4-(N,N-dimethylamino)benzophenone, and n-butyl 4-(N,N-dimethylamino)benzoate.

The amine-based reducing agents can be broadly classified into aromatic amines and aliphatic amines. In the present invention, the amine-based reducing agents may be aromatic amines or aliphatic amines.

The aromatic amines may be, for example, known aromatic secondary amines or aromatic tertiary amines. Examples of the aromatic secondary amines or aromatic tertiary amines include N,N-bis(2-hydroxyethyl)-3,5-dimethylaniline, N,N-di(2-hydroxyethyl)-p-toluidine, N,N-bis(2-hydroxyethyl)-3,4-dimethylaniline, N,N-bis(2-hydroxyethyl)-4-ethylaniline, N,N-bis(2-hydroxyethyl)-4-isopropylaniline, N,N-bis(2-hydroxyethyl)-4-t-butylaniline, N,N-bis(2-hydroxyethyl)-3,5-di-isopropylaniline, N,N-bis(2-hydroxyethyl)-3,5-di-t-butylaniline, N,N-dimethylaniline, N,N-dimethyl-p-toluidine, N,N-dimethyl-m-toluidine, N,N-diethyl-p-toluidine, N,N-dimethyl-3,5-dimethylaniline, N,N-dimethyl-3,4-dimethylaniline, N,N-dimethyl-4-ethylaniline, N,N-dimethyl-4-isopropylaniline, N,N-dimethyl-4-t-butylaniline, and N,N-dimethyl-3,5-di-t-butylaniline.

Examples of the aliphatic amines include aliphatic primary amines such as n-butylamine, n-hexylamine, and n-octylamine; aliphatic secondary amines such as diisopropylamine, dibutylamine, and N-methylethanolamine; and aliphatic tertiary amines such as N-methyldiethanolamine, N-ethyldiethanolamine, N-n-butyldiethanolamine, N-lauryldiethanolamine, 2-(dimethylamino)ethyl (meth)acrylate, N-methyldiethanolamine di(meth)acrylate, N-ethyldiethanolamine di(meth)acrylate, triethanolamine tri(meth)acrylate, triethanolamine, trimethylamine, triethylamine, and tributylamine.

In view of curability and storage stability, preferred among these photopolymerization accelerators are aminobenzoic acid ester compounds, and aliphatic tertiary amines, more preferably ethyl 4-(N,N-dimethylamino)benzoate, N-methyldiethanolamine, and triethanolamine.

The photopolymerization accelerator (D-1) may be incorporated alone, or two or more thereof may be used in combination.

In view of curability and storage stability, the content of photopolymerization accelerator (D-1) is preferably 0.01 to 10 parts by mass, more preferably 0.05 to 5 parts by mass, even more preferably 0.1 to 3 parts by mass with respect to total 100 parts by mass of polymerizable monomer (A). It is easier to provide adequate adhesive properties when the content of photopolymerization accelerator (D-1) is 0.01 parts or more by mass, whereas a content of 10 parts or less by mass facilitates maintaining the working time margin.

### <Chemical Polymerization Accelerator (D-2)>

A certain preferred embodiment is, for example, a curable dental composition in which the polymerization accelerator (D) comprises a chemical polymerization accelerator (D-2).

The chemical polymerization accelerator (D-2) used in a curable dental composition of the present invention can be broadly classified into chemical polymerization accelerator (D-2a), which is suitably used when the polymerizable monomer (A) does not comprise a polymerizable monomer having an acidic group (A-2) (hereinafter, also referred to simply as "chemical polymerization accelerator (D-2a)"), and chemical polymerization accelerator (D-2b), which is suitably used when the polymerizable monomer (A) comprises a polymerizable monomer having an acidic group (A-2) (hereinafter, also referred to simply as "chemical polymerization accelerator (D-2b)").

The chemical polymerization accelerator (D-2) can be selected according to the type of polymerizable monomer, and may comprise either one or both of chemical polymerization accelerator (D-2a), which is suitably used when not containing a polymerizable monomer having an acidic group (A-2), and chemical polymerization accelerator (D-2b), which is suitably used when containing a polymerizable monomer having an acidic group (A-2).

Note that copper compounds can be suitably used for both situations where the polymerizable monomer (A) comprises or does not comprise a polymerizable monomer having an acidic group (A-2). Accordingly, copper compounds are presented as examples for both chemical polymerization accelerator (D-2a) and chemical polymerization accelerator (D-2b).

### · Chemical Polymerization Accelerator (D-2a)

Examples of the chemical polymerization accelerator (D-2a) in the curable dental composition include aromatic amines having no electron withdrawing group on the aromatic ring, period 4 transition metal compounds, transition metal compounds outside of period 4, and thiourea compounds. Specific examples are as follows.

Examples of aromatic amines having no electron withdrawing group on the aromatic ring include N,N-bis(2-hydroxyethyl)-3,5-dimethylaniline, N,N-bis(2-hydroxyethyl)-p-toluidine, N,N-bis(2-hydroxyethyl)-3,4-dimethylaniline, N,N-bis(2-hydroxyethyl)-4-ethylaniline, N,N-bis(2-hydroxyethyl)-4-isopropylaniline, N,N-bis(2-hydroxyethyl)-4-t-butylaniline, N,N-bis(2-hydroxyethyl)-3,5-di-isopropylaniline, N,N-bis(2-hydroxyethyl)-3,5-di-t-butylaniline, N,N-dimethylaniline, N,N-dimethyl-p-toluidine, N,N-dimethyl-m-toluidine, N,N-diethyl-p-toluidine, N,N-dimethyl-3,5-dimethylaniline, N,N-dimethyl-3,4-dimethylaniline, N,N-dimethyl-4-ethylaniline, N,N-dimethyl-4-isopropylaniline, N,N-dimethyl-4-t-butylaniline, and N,N-dimethyl-3,5-di-t-butylaniline.

The period 4 transition metal compounds may be any of vanadium compounds, copper compounds, and period 4 transition metal compounds excluding vanadium and copper.

In view of polymerization accelerating effect, preferred as chemical polymerization accelerator (D-2a) in the present invention are vanadium compounds and copper compounds.

Examples of the vanadium compounds include vanadyl acetylacetonate, vanadyl stearate, vanadium naphthenate, vanadium benzoylacetonate, vanadyl oxalate, bis(maltolato)oxovanadium(IV), oxobis(1-phenyl-1,3-butanedionate)vanadium(IV), vanadium(V) oxytriisopropoxide, ammonium metavanadate, sodium metavanadate, vanadium(V) oxide, vanadium(IV) oxide, and vanadyl(IV) sulfate. In view of properties such as the solubility in solvent, preferred are vanadyl acetylacetonate, and bis(maltolato)oxovanadium(IV), more preferably vanadyl acetylacetonate and bis(maltolato)oxovanadium(IV).

Preferably, the copper compounds are compounds that are soluble in radical polymerizable monomers.

Specific examples of the copper compounds include copper(II) carboxylates (for example, copper(II) acetate, copper(II) benzoate, copper(II) isobutyrate, copper(II) gluconate, copper(II) citrate, copper(II) phthalate, copper(II) tartarate, copper(II) oleate, copper(II) octylate, copper(II) octenate, copper(II) naphthenate, copper(II) acrylate, copper(II) methacrylate, copper(II) 4-cyclohexylbutyrate); copper β-diketones (for example, copper(II) acetylacetonate, copper(II) trifluoroacetylacetonate, copper(II) hexafluoroacetylacetonate, copper(II) 2,2,6,6-tetramethyl-3,5-heptanedionate, copper(II) benzoylacetonate); copper β-ketoesters (for example, copper(II) ethyl acetoacetate); copper alkoxides (for example, copper(II) methoxide, copper(II) ethoxide, copper(II) isopropoxide, copper(II) 2-(2-butoxyethoxy)ethoxide, copper(II) 2-(2-methoxyethoxy)ethoxide); copper dithiocarbamates (for example, copper(II) dimethyldithiocarbamates); salts of copper and inorganic acids (for example, copper(II) sulfate, copper(II) nitrate); copper(II) chloride, and copper disodium ethylenediaminetetraacetate; copper procetonate, and copper complexes.

In view of solubility and reactivity to radical polymerizable monomers, preferred are copper(II) carboxylates, copper(II) β-diketones, and copper(II) β-ketoesters, more preferably copper(II) acetate, and copper(II) acetylacetonate.

Examples of other period 4 transition metal compounds include scandium isopropoxide, iron(III) ethoxide, titanium methoxide, titanium ethoxide, titanium isopropoxide, titanium butoxide, titanium hydroxide, and titanium fluoride.

In view of enhanced polymerization accelerating effect, preferred among the period 4 transition metal compounds are vanadyl(IV) acetylacetonate, and bis(maltolato)oxovanadium(IV), more preferably vanadyl(IV) acetylacetonate.

Examples of the transition metal compounds outside of period 4 include strontium carbonate, strontium hydroxide, strontium ethoxide, tin(II) methoxide, indium ethoxide, actinium ethoxide, yttrium isopropoxide, lanthanum methoxide, lanthanum ethoxide, lanthanum isopropoxide, lanthanum butoxide, lanthanum hydroxide, lanthanum carbonate, lanthanum fluoride, cerium isopropoxide, praseodymium isopropoxide, promethium isopropoxide, neodymium isopropoxide, samarium isopropoxide, europium isopropoxide, gadolinium isopropoxide, terbium ethoxide, terbium methoxide, dysprosium isopropoxide, holmium isopropoxide, erbium isopropoxide, thulium isopropoxide, ytterbium isopropoxide, zirconium ethoxide, zirconium isopropoxide, zirconium butoxide, tungsten(IV) methoxide, tungsten(IV) isopropoxide, and tungsten(IV) butoxide.

Examples of the thiourea compounds include thiourea, methylthiourea, ethylthiourea, ethylenethiourea, N,N'-dimethylthiourea, N,N'-diethylthiourea, N,N'-di-n-propylthiourea, N,N'-dicyclohexylthiourea, trimethylthiourea, triethylthiourea, tri-n-propylthiourea, tricyclohexylthiourea, tetramethylthiourea, tetraethylthiourea, tetra-n-propylthiourea, tetracyclohexylthiourea, 1-(2-pyridyl)-2-thiourea, (6-methyl-pyridin-2-yl)thiourea, and 4,4-dimethylethylenethiourea. In view of properties such as the solubility in solvent and storage stability, preferred are 1-(2-pyridyl)-2-thiourea, (6-methyl-pyridin-2-yl)thiourea, and 4,4-dimethylethylenethiourea.

The chemical polymerization accelerator (D-2a) may be incorporated alone, or two or more thereof may be used in combination.

In view of curability and storage stability, the content of chemical polymerization accelerator (D-2a) is preferably 0.001 to 20 parts by mass, more preferably 0.005 to 10 parts by mass, even more preferably 0.025 to 5 parts by mass with respect to total 100 parts by mass of polymerizable monomer (A).

### · Chemical Polymerization Accelerator (D-2b)

Examples of the chemical polymerization accelerator (D-2b) in the curable dental composition include copper compounds, aromatic sulfinic acids and salts thereof, benzotriazole compounds, benzoimidazole compounds, bromides, and sulfur-containing reducing inorganic compounds. Specific examples are as follows.

Preferably, the copper compounds are compounds that are soluble in the polymerizable monomer components. Specific examples of such compounds include copper(II) carboxylates (for example, copper(II) acetate, copper(II) benzoate, copper(II) isobutyrate, copper(II) gluconate, copper(II) citrate, copper(II) phthalate, copper(II) tartarate, copper(II) oleate, copper(II) octylate, copper(II) octenate, copper(II) naphthenate, copper(II) acrylate, copper(II) methacrylate, copper(II) 4-cyclohexylbutyrate); copper β-diketones (for example, copper(II) acetylacetonate, copper(II) trifluoroacetylacetonate, copper(II) hexafluoroacetylacetonate, copper(II) 2,2,6,6-tetramethyl-3,5-heptanedionate, copper(II) benzoylacetonate); copper β-ketoesters (for example, copper(II) ethyl acetoacetate); copper alkoxides (for example, copper(II) methoxide, copper(II) ethoxide, copper(II) isopropoxide, copper(II) 2-(2-butoxyethoxy)ethoxide, copper(II) 2-(2-methoxyethoxy)ethoxide); copper dithiocarbamates (for example, copper(II) dimethyldithiocarbamate); salts of copper and inorganic acids (for example, copper(II) sulfate, copper(II) nitrate); copper(II) chloride, and copper disodium ethylenediaminetetraacetate; copper procetonate, and copper complexes.

In view of solubility and reactivity to the polymerizable monomers, preferred are copper(II) carboxylates, copper(II) β-diketones, and copper(II) β-ketoesters, more preferably copper(II) acetate, and copper(II) acetylacetonate.

Examples of the aromatic sulfinic acids and salts thereof include benzenesulfinic acid, p-toluenesulfinic acid, o-toluenesulfinic acid, ethylbenzenesulfinic acid, decylbenzenesulfinic acid, dodecylbenzenesulfinic acid, 2,4,6-trimethylbenzenesulfinic acid, 2,4,6-triisopropylbenzenesulfinic acid, chlorobenzenesulfinic acid, naphthalenesulfinic acid, and lithium salts, sodium salts, potassium salts, rubidium salts, cesium salts, magnesium salts, calcium salts, strontium salts, iron salts, zinc salts, ammonium salts, tetramethylammonium salts, and tetraethylammonium salts of these. In view of polymerizability and storage stability of the composition, preferred among these are lithium salts, sodium salts, potassium salts, magnesium salts, and calcium salts of 2,4,6-trimethylbenzenesulfinic acid and 2,4,6-triisopropylbenzenesulfinic acid, more preferably lithium salts, sodium salts, potassium salts, magnesium salts, and calcium salts of 2,4,6-triisopropylbenzenesulfinic acid.

Preferably, the aromatic sulfinic acids and salts thereof are at least partially dispersed in powder form within the composition. With the aromatic sulfinic acids and salts thereof being dispersed in powder form, it is easier to provide a longer working time margin for a curable dental composition of the present invention, and, when the composition is applied to moist materials such as the tooth structure, the aromatic sulfinic acids and salts thereof dissolve in the water on the surface of the moist material, making it possible to more greatly enhance polymerization both at the bonding interface and within the resin-impregnated layer. When the aromatic sulfinic acids and salts thereof are dispersed as powder, it is preferable that the aromatic sulfinic acids and salts thereof have a solubility in water of 1 mg/100 mL or more at ordinary temperature (25°C). A solubility of 1 mg/100 mL or more facilitates sufficient dissolution of aromatic sulfinic acids and salts thereof in the water on moist materials at the bonding interface in applications where a curable dental composition of the present invention is applied to moist materials, facilitating the effect of dispersing as powder. In view of resistance to settling, the aromatic sulfinic acids and salts thereof have an average particle diameter of preferably 500 µm or less, more preferably 100 µm or less, even more preferably 50 µm or less. In view of preventing excessive increase in the specific surface area of the powder and facilitating in maintaining good ease of handling for the curable dental composition, the average particle diameter is preferably 0.01 µm or more. That is, when dispersed as powder, the aromatic sulfinic acids and salts thereof have an average particle diameter ranging from preferably 0.01 to 500 µm, more preferably from 0.01 to 100 µm. The average particle diameter can be measured in the same manner as the average particle diameter of filler (B).

The shape of the aromatic sulfinic acids and salts thereof dispersed as powder is not particularly limited, and may be any of various shapes such as spherical, needle-shape, plate-like, and crushed-shape. Fine powders of aromatic sulfinic acid salts can be prepared using known methods such as pulverization and freeze drying.

In view of working time margin and adhesive properties, the content of the aromatic sulfinic acids and salts thereof is preferably 0.1 to 5 parts by mass, more preferably 0.2 to 4 parts by mass, even more preferably 0.5 to 3 parts by mass with respect to total 100 parts by mass of the polymerizable monomer (A) in an adhesive composition of the present invention. A content of less than 0.1 parts by mass may cause a decrease in adhesive properties, whereas a content of more than 5 parts by mass may result in shorter working time margins.

The benzotriazole compounds and benzoimidazole compounds are represented by the following general formulae (2) and (3), respectively.

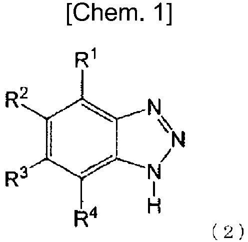

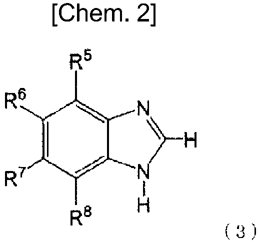

In the general formulae (2) and (3), R¹ to R⁸ each independently represent a hydrogen atom, a hydroxyl group, an alkyl group, an aryl group, an alkoxy group, an alkenyl group, an aralkyl group, or a halogen atom.

The alkyl groups represented by R¹ to R⁸ may be linear, branched, or cyclic, and are preferably ones having 1 to 10 carbon atoms. Examples of the alkyl groups include methyl groups, ethyl groups, n-propyl groups, isopropyl groups, cyclopropyl groups, n-butyl groups, isobutyl groups, s-butyl groups, tert-butyl groups, cyclobutyl groups, n-pentyl groups, isopentyl groups, neopentyl groups, tert-pentyl groups, cyclopentyl groups, n-hexyl groups, isohexyl groups, cyclohexyl groups, n-heptyl groups, cycloheptanyl groups, n-octyl groups, 2-ethylhexyl groups, cyclooctyl groups, n-nonyl groups, cyclononyl groups, and n-decyl groups. Particularly preferred among these are methyl groups and ethyl groups.

The aryl groups represented by R¹ to R⁸ are preferably ones having 6 to 10 carbon atoms. Examples of the aryl groups include phenyl groups, naphthyl groups, and anthryl groups.

The alkoxy groups represented by R¹ to R⁸ may be linear, branched, or cyclic, and are preferably ones having 1 to 8 carbon atoms. Examples of the alkoxy groups include methoxy groups, ethoxy groups, n-propoxy groups, isopropoxy groups, n-butoxy groups, tert-butoxy groups, n-hexyloxy groups, cyclohexyloxy groups, n-octyloxy groups, and 2-ethylhexyloxy groups.

The alkenyl groups represented by R¹ to R⁸ may be linear, branched, or cyclic, and are preferably ones having 1 to 6 carbon atoms. Examples of the alkenyl groups include vinyl groups, allyl groups, methylvinyl groups, propenyl groups, butenyl groups, pentenyl groups, hexenyl groups, cyclopropenyl groups, cyclobutenyl groups, cyclopentenyl groups, and cyclohexenyl groups.

The aralkyl groups represented by R¹ to R⁸ are preferably alkyl groups (particularly, C1 to C10 alkyl groups) substituted with aryl groups (particularly, C6 to C10 aryl groups). Examples include benzyl groups.

Examples of the halogen atoms represented by R¹ to R⁸ include a fluorine atom, a chlorine atom, a bromine atom, and an iodine atom.

R¹ to R⁸ are preferably hydrogen atoms or methyl groups.

Only one type of benzotriazole compound or benzoimidazole compound may be used alone, or more than one type of benzotriazole compound or benzoimidazole compound may be used in combination. Examples of the benzotriazole compounds and benzoimidazole compounds include 1H-benzotriazole, 5-methyl-1H-benzotriazole, 5,6-dimethyl-1H-benzotriazole, benzoimidazole, 5-methylbenzoimidazole, and 5,6-dimethylbenzoimidazole. In view of working time margin, preferred are 1 H-benzotriazole, and 5-methyl-1H-benzotriazole.

Examples of the bromides include zinc bromide, potassium bromide, sodium bromide, calcium bromide, indium bromide, ammonium bromide, and tetrabutylammonium bromide. Particularly preferred among these are zinc bromide, ammonium bromide, and tetrabutylammonium bromide.

Examples of the sulfur-containing reducing inorganic compounds include sulfites, bisulfites, pyrosulfites, thiosulfates, thionates, and dithionites. Preferred among these are sulfites and bisulfites. Specific examples include sodium sulfite, potassium sulfite, calcium sulfite, ammonium sulfite, sodium bisulfite, and potassium bisulfite.

Preferably, the sulfur-containing reducing inorganic compounds are at least partially dispersed in powder form within the composition. With the sulfur-containing reducing inorganic compounds being dispersed in powder form, it is possible to ensure a longer working time margin for a curable dental composition of the present invention, and, when the composition is applied to the tooth structure, the sulfur-containing reducing inorganic compounds dissolve in the water on the surface of the tooth structure, making it possible to more greatly enhance polymerization both at the bonding interface and within the resin-impregnated layer. When the sulfur-containing reducing inorganic compounds are dispersed as powder, it is preferable that the sulfur-containing reducing inorganic compounds have a solubility in water of 1 mg/100 mL or more at ordinary temperature (25°C). A solubility of 1 mg/100 mL or more facilitates sufficient dissolution of sulfur-containing reducing inorganic compounds in the water on tooth structure at the bonding interface when a curable dental composition of the present invention is applied to tooth structure, facilitating the effect of dispersing as powder. In view of resistance to settling, the sulfur-containing reducing inorganic compounds have an average particle diameter of preferably 500 µm or less, more preferably 100 µm or less, even more preferably 50 µm or less. In view of preventing excessive increase in the specific surface area of the powder and facilitating in maintaining good ease of handling for the curable dental composition, the average particle diameter is preferably 0.01 µm or more. That is, when dispersed as powder, the average particle diameter ranges preferably from 0.01 to 500 µm, more preferably from 0.01 to 100 µm. The average particle diameter can be measured in the same manner as the average particle diameter of filler (B).

The shape of the sulfur-containing reducing inorganic compounds dispersed as powder is not particularly limited, and may be any of various shapes such as spherical, needle-shape, plate-like, and crushed-shape. Fine powders of sulfur-containing reducing inorganic compounds can be prepared using known methods such as pulverization and freeze drying.

The chemical polymerization accelerator (D-2b) may be incorporated alone, or two or more thereof may be used in combination.

In view of curability and storage stability, the content of chemical polymerization accelerator (D-2b) is preferably 0.001 to 40 parts by mass, more preferably 0.005 to 20 parts by mass, even more preferably 0.025 to 10 parts by mass with respect to total 100 parts by mass of polymerizable monomer (A).

When a curable dental composition of the present invention comprises a chemical polymerization initiator (C-2) and a chemical polymerization accelerator (D-2), it is preferable in view of storage stability that the composition be packaged into a product form with two, three, or more separate packs to prevent reactions among its components prior to use. In this case, it is preferable to formulate the chemical polymerization initiator (C-2) separately from the thiourea compounds or aromatic sulfinic acids and salts thereof.

In the case of a two-pack type curable dental composition, it is preferable to incorporate the polymerizable monomer (A) and the filler (B) in both the first and second agents to prepare a two-paste composition.

A certain embodiment is, for example, a curable dental composition that comprises a first agent and a second agent, wherein:
the first agent comprises a polymerizable monomer (A), a filler (B), and a chemical polymerization initiator (C-2),
the second agent comprises a polymerizable monomer (A), a filler (B), and a chemical polymerization accelerator (D-2), and
the first agent and/or the second agent comprise a rheology modifier (E).

The rheology modifier (E) may be contained in only the first agent or only the second agent. However, in view of the storage stability of each agent, it is preferable to comprise the rheology modifier (E) in both the first agent and the second agent.

### [Rheology Modifier (E)]

A curable dental composition of the present invention must comprise a rheology modifier (E) to achieve both superior paste flowability and excellent shape retention in excess cement for easy removal of excess cement by preliminary irradiation.

The rheology modifier (E) refers to a component that can alter the viscoelasticity of the curable dental composition. By incorporating the rheology modifier (E), the viscosity of the curable dental composition decreases from its viscosity at rest when pressure is applied, and return to the viscosity at rest when the pressure is released.

Examples of the rheology modifier (E) include an inorganic fine particle (E-1) having an average particle diameter of less than 1 µm (hereinafter, referred to as inorganic fine particle (E-1)), a (meth)acrylic compound (E-2) having a weight-average molecular weight of 1,000 to 80,000, and a weight-average molecular weight per polymerizable group of 1,250 or more and less than 20,000 (hereinafter, referred to as (meth)acrylic compound (E-2)), a hydrophilic polymer (E-3),and cellulose nanofibers.

Components included in filler (B) are excluded from the rheology modifier (E).

Examples of the components of inorganic fine particle (E-1) include the following, provided that the average particle diameter is less than 1 µm:
various types of glass (containing silica as a main component, and, optionally, oxides of elements such as heavy metals, boron, and aluminum), including, for example, glass powders of common compositions, such as fused silica, quartz, soda-lime-silica glass, E glass, C glass, and borosilicate glass (PYREX^{®} glass), and dental glass powders such as barium glass (GM27884 and 8235 manufactured by SCHOTT, and E-2000 and E-3000 manufactured by ESSTECH), strontium·borosilicate glass (E-4000 manufactured by ESSTECH), lanthanum glass-ceramic (GM31684 manufactured by SCHOTT), and fluoroaluminosilicate glass (GM35429, G018-091, and G018-117 manufactured by SCHOTT); and
various types of ceramics, composite oxides (such as silica-titania, and silica-zirconia), diatomaceous earth, kaolin, clay minerals (such as montmorillonite), activated earth, synthetic zeolite, mica, calcium fluoride, ytterbium fluoride, yttrium fluoride, core-shell structured calcium fluoride with silica coating, core-shell structured ytterbium fluoride with silica coating, core-shell structured yttrium fluoride with silica coating, calcium phosphate, barium sulfate, zirconium dioxide, titanium dioxide, hydroxyapatite, core-shell structured calcium phosphate with silica coating, core-shell structured barium sulfate with silica coating, core-shell structured zirconium dioxide with silica coating, core-shell structured titanium dioxide with silica coating, and core-shell structured hydroxyapatite with silica coating.

In view of strength and other properties, preferred among these are various types of glass, composite oxides (such as silica-titania, and silica-zirconia), core-shell structured calcium fluoride with silica coating, core-shell structured ytterbium fluoride with silica coating, core-shell structured yttrium fluoride with silica coating, core-shell structured calcium phosphate with silica coating, core-shell structured barium sulfate with silica coating, core-shell structured zirconium dioxide with silica coating, core-shell structured titanium dioxide with silica coating, and core-shell structured hydroxyapatite with silica coating.

The inorganic fine particle (E-1) used in the present invention has an average particle diameter of less than 1 µm. In view of the flowability of the curable dental composition under applied external force, and facilitating the unique viscosity behavior where the viscosity greatly decreases under applied pressure compared to when at rest and quickly returns to the resting viscosity upon relieving the stress, as well as the mechanical strength and transparency of the cured product, the average particle diameter of inorganic fine particle (E-1) is more preferably 0.001 to 0.5 µm, even more preferably 0.01 to 0.3 µm.

A certain preferred embodiment is, for example, a curable dental composition in which the rheology modifier (E) comprises an inorganic fine particle (E-1) having an average particle diameter of less than 1 µm.

Another certain preferred embodiment is, for example, a curable dental composition in which the inorganic fine particle (E-1) has an average particle diameter of 7 to 200 nm.

In view of facilitating the unique viscosity behavior, the inorganic fine particle (E-1) may have an average particle diameter of 100 nm or less.

The average particle diameter of rheology modifier (E) can be measured by a laser diffraction scattering method or electron microscopy. The laser diffraction scattering method is as described above.

In electron microscopy, for example, particles may be photographed with an electron microscope (Model S-4000, manufactured by Hitachi Ltd.), and the size of particles (at least 200 particles) observed in a unit field of the captured image may be measured using image-analyzing particle-size-distribution measurement software (Mac-View, manufactured by Mountech Co., Ltd.). Here, the particle diameter is determined as an arithmetic mean value of the maximum and minimum lengths of particles, and the average primary particle diameter is calculated from the number of particles and the particle diameter.

The inorganic fine particle (E-1) may be spherical or irregular in shape, for example.

In a curable dental composition of the present invention, in view of the storage stability of the curable dental composition and the flowability under applied external force, the inorganic fine particle (E-1) preferably comprises a spherical inorganic fine particle (E-1a), more preferably a combination of spherical inorganic fine particle (E-1a) and irregularly shaped inorganic fine particle (E-1b).

### <Spherical Inorganic Fine Particle (E-1a)>

The spherical inorganic fine particle (E-1a) is a filler representing particles that appear round in shape when observed in a unit field of an electron micrograph, and that have an average uniformity of 0.6 or more as calculated by dividing the diameter of a particle perpendicular to its maximum diameter by the maximum diameter.

In view of providing even superior sliding effect under applied external force, the spherical inorganic fine particle (E-1a) in a curable dental composition of the present invention has an average uniformity of preferably 0.7 or more, more preferably 0.8 or more.

The filler material is not particularly limited because the spherical inorganic fine particle (E-1a), by being spherical, exhibits a sliding effect under applied external force, and produces an effect that mitigates interference between fillers, leading to paste flowability under applied external force.

A certain embodiment is, for example, a curable dental composition in which the spherical inorganic fine particle (E-1a) is an inorganic filler.

Another preferred embodiment is, for example, a curable dental composition in which the spherical inorganic fine particle (E-1a) is perfectly spherical nanosilica.

Here, the term "perfectly spherical" denotes an average uniformity of preferably 0.9 or more, more preferably 0.95 or more.

With perfectly spherical nanosilica, the silanol groups on the particle surface of silica form a crosslinked structure by hydrogen bonding, and produce a sliding effect. This helps provide the necessary unique viscosity behavior, enabling a shorter time T for viscosity V3 to reach 10,000 Pa·s after the shear rate is switched from D2 back to D1.

The spherical inorganic fine particle (E-1a) used in the present invention does not necessarily need to be produced or acquired as a singular particle, and may be one obtained by mixing two or more fillers of different average particle diameters or different components, provided that the average particle diameter of each filler falls within the foregoing ranges.

### <Irregularly Shaped Inorganic Fine Particle (E-1b)>

The irregularly shaped inorganic fine particle (E-1b) is a filler representing particles that do not appear round in shape when observed in a unit field of an electron micrograph, and that have an average uniformity of less than 0.6 as calculated by dividing the diameter of a particle perpendicular to its maximum diameter by the maximum diameter.

The irregularly shaped inorganic fine particle (E-1b) does not necessarily need to be produced or acquired as a singular particle, and may be one obtained by mixing two or more fillers of different average particle diameters or different components, provided that the average particle diameter of each filler falls within the foregoing ranges.

In view of flowability under applied external force, the shape retention at rest, and the mechanical strength of the cured product, the content of the inorganic fine particle (E-1) in a curable dental composition of the present invention is preferably 0.1 to 200 parts by mass with respect to total 100 parts by mass of polymerizable monomer (A). In view of advantages such as achieving an even shorter time for the dropped viscosity to quickly return to the resting viscosity upon relieving the stress, and providing even superior paste flowability under applied pressure, the content of inorganic fine particle (E-1) is more preferably 0.5 to 100 parts by mass, even more preferably 1 to 50 parts by mass.

In certain embodiments, in view of superior shape retention at rest and superior mechanical strength of the cured product, the content of the inorganic fine particle (E-1) in a curable dental composition of the present invention is preferably 0.1 to 50 mass% in total 100 mass% of the composition. In view of advantages such as achieving an even shorter time for the dropped viscosity to quickly return to the resting viscosity upon relieving the stress, and providing even superior paste flowability under applied pressure, the content of inorganic fine particle (E-1) is more preferably 0.2 to 30 mass%, even more preferably 1 to 10 mass%.

The inorganic fine particle (E-1) in the present invention may be one that is surface-treated with a hydrophobizing agent (x-1), in order to improve its dispersibility in the polymerizable monomers. The hydrophobizing agent (x-1) is not particularly limited, as long as the inorganic fine particle can be hydrophobized and reduce hydrophilic interactions with the polymerizable monomers. Any known surface treatment agents capable of imparting hydrophobic properties can be used without limitation.

The hydrophobizing agent (x-1) used to hydrophobize the inorganic fine particle (E-1) may be the same hydrophobizing agent (x-1) used for the filler (B).

When the curable dental composition comprises the filler (B) and the inorganic fine particle (E-1), and the filler (B) and inorganic fine particle (E-1) are both hydrophobized, the hydrophobizing agent (x-1) used to hydrophobize the inorganic fine particle (E-1) may be the same or different from the hydrophobizing agent (x-1) used to hydrophobize the filler (B).

In certain embodiments, the mixing ratio of filler (B) and inorganic fine particle (E-1) is preferably 100:0.1 to 100:50, more preferably 100:0.15 to 100:25, even more preferably 100:0.2 to 100:15.

The content of rheology modifier (E) can be appropriately changed according to the material type, provided that it remains within a range that allows the present invention to exhibit its effects. For example, when the rheology modifier (E) is solely irregularly shaped inorganic fine particle (E-1b), the mixing ratio of filler (B) and irregularly shaped inorganic fine particle (E-1b) may range from 100:0.2 to 100:10, or 100:0.5 to 100:8.

The (meth)acrylic compound (E-2) can be broadly classified into urethanized (meth)acrylic compound (E-2a), and (meth)acrylic compound having no urethane backbone (E-2b).

In view of allowing for easy introduction of (meth)acryl groups, and achieving the unique viscosity behavior where the viscosity greatly decreases under applied pressure compared to when at rest, and quickly returns to the resting viscosity upon relieving the stress, as well as producing an effect that reduces the polymerization shrinkage stress, the (meth)acrylic compound (E-2) is preferably a urethanized (meth)acrylic compound (E-2a). As an example, the urethanized (meth)acrylic compound (E-2a) can be easily synthesized through an addition reaction between a polyol having a polymer backbone, a compound having an isocyanate group (-NCO), and a (meth)acrylic compound having a hydroxyl group (-OH) (these will be described later). The urethanized (meth)acrylic compound (E-2a) can also be easily synthesized by allowing lactone or alkylene oxide to undergo a ring-opening addition reaction with a (meth)acrylic compound having a hydroxyl group, and causing the resulting compound with a terminal hydroxyl group to undergo an addition reaction with a compound having an isocyanate group.

The (meth)acrylic compound having no urethane backbone (E-2b) can be obtained, for example, through a dehydrocondensation reaction of (meth)acrylic acid with a polymer of a hydroxyl group-containing monomer.

In the polymerizable monomer having no acidic group (A-1), (meth)acrylic compounds with a weight-average molecular weight of 1,000 to 80,000, and a weight-average molecular weight per polymerizable group of 1,250 or more and less than 20,000 are categorized herein as (meth)acrylic compound (E-2), regardless of their solubility in 25°C water.

### <Urethanized (Meth)Acrylic Compound (E-2a)>

The urethanized (meth)acrylic compound (E-2a) is preferably a (meth)acrylate that comprises a structure (polymer backbone) selected from the group consisting of a polyester, a polycarbonate, a polyurethane, a polyether, a poly-conjugated diene, and a hydrogenated poly-conjugated diene, in addition to a urethane bond. More preferably, the urethanized (meth)acrylic compound (E-2a) is a (meth)acrylate that comprises, within a single molecule, at least one polyol moiety selected from the group consisting of a polyester, a polycarbonate, a polyurethane, a polyether, a poly-conjugated diene, and a hydrogenated poly-conjugated diene each having a structure derived from a C4 to C18 aliphatic diol unit having a branched structure.

In these structures, examples of the polyester include copolymers of dicarboxylic acids (aromatic dicarboxylic acids such as phthalic acid and isophthalic acid, and unsaturated aliphatic dicarboxylic acids such as maleic acid) and aliphatic diols having 2 to 18 carbon atoms, copolymers of dicarboxylic acids (saturated aliphatic dicarboxylic acids such as adipic acid and sebacic acid) and aliphatic diols having 2 to 18 carbon atoms, β-propiolactone polymers, γ-butyrolactone polymers, δ-valerolactone polymers, ε-caprolactone polymers, and copolymers of these. Preferred are copolymers of dicarboxylic acids (aromatic dicarboxylic acids such as phthalic acid and isophthalic acid, and unsaturated aliphatic dicarboxylic acids such as maleic acid) and aliphatic diols having 2 to 12 carbon atoms, and copolymers of dicarboxylic acids (saturated aliphatic dicarboxylic acids such as adipic acid and sebacic acid) and aliphatic diols having 2 to 12 carbon atoms.

Examples of the polycarbonate include polycarbonates derived from C2 to C18 aliphatic diols, polycarbonates derived from bisphenol A, and polycarbonates derived from C2 to C18 aliphatic diols and bisphenol A. Preferred are polycarbonates derived from C2 to C12 aliphatic diols, polycarbonates derived from bisphenol A, and polycarbonates derived from C2 to C12 aliphatic diols and bisphenol A.

Examples of the polyurethane include polymers of C2 to C18 aliphatic diols and C1 to C18 diisocyanates. Preferred are polymers of C2 to C12 aliphatic diols and C1 to C12 diisocyanates.

Examples of the polyether include polyethylene glycol, polypropylene glycol, polybutylene glycol, and poly(1-methylbutylene glycol).

Examples of the poly-conjugated diene and hydrogenated poly-conjugated diene include 1,4-polybutadiene, 1,2-polybutadiene, polyisoprene, poly(butadiene-isoprene), poly(butadiene-styrene), poly(isoprene-styrene), polyfarnesene, and hydrogenated products of these. In view of superior mechanical strength and water resistance, preferred among these are the polyester, polycarbonate, and poly-conjugated diene structures. Polyols with the aforementioned polymer backbones can be used for the production of urethanized (meth)acrylic compound (E-2a).

Examples of the compound having an isocyanate group include hexamethylene diisocyanate (HDI), tolylene diisocyanate (TDI), xylylene diisocyanate (XDI), diphenylmethane diisocyanate (MDI), isophorone diisocyanate (IPDI), trimethylhexamethylene diisocyanate (TMHMDI), tricyclodecane diisocyanate (TCDDI), and adamantane diisocyanate (ADI).

Examples of the (meth)acrylic compound having a hydroxyl group include hydroxy (meth)acrylate compounds such as 2-hydroxyethyl (meth)acrylate, 2-hydroxypropyl (meth)acrylate, 2-hydroxybutyl (meth)acrylate, 6-hydroxyhexyl (meth)acrylate, 10-hydroxydecyl (meth)acrylate, 3-chloro-2-hydroxypropyl (meth)acrylate, 2-hydroxy-3-phenoxypropyl (meth)acrylate, glycerin mono(meth)acrylate, 2-hydroxy-3-acryloyloxypropyl (meth)acrylate, 2,2-bis[4-[3-(meth)acryloyloxy-2-hydroxypropoxy]phenyl]propane, 1,2-bis[3-(meth)acryloyloxy-2-hydroxypropoxy]ethane, pentaerythritol tri(meth)acrylate, and tri or tetra(meth)acrylates of dipentaerythritol; and hydroxy(meth)acrylamide compounds such as N-hydroxyethyl(meth)acrylamide, and N,N-bis(2-hydroxyethyl)(meth)acrylamide.

Examples of the C4 to C18 aliphatic diols having a branched structure include 2-methyl-1,3-propanediol, 2,2-diethyl-1,3-propanediol, 1,3-butanediol, 2-methyl-1,4-butanediol, neopentyl glycol, 3-methyl-1,5-pentanediol, 2-methyl-1,8-octanediol, 2,7-dimethyl-1,8-octanediol, 2-methyl-1,9-nonanediol, 2,8-dimethyl-1,9-nonanediol, 2-methyl-1,10-decanediol, 2,9-dimethyl-1,10-decanediol, 2-methyl-1,11-undecanediol, 2,10-dimethyl-1,11-undecanediol, 2-methyl-1,12-dodecanediol, 2,11-dimethyl-1,12-dodecanediol, 2-methyl-1,13-tridecanediol, 2,12-dimethyl-1,13-tridecanediol, 2-methyl-1,14-tetradecanediol, 2,13-dimethyl-1,14-tetradecanediol, 2-methyl-1,15-pentadecanediol, 2,14-dimethyl-1,15-pentadecanediol, 2-methyl-1,16-hexadecanediol, and 2,15-dimethyl-1,16-hexadecanediol. In view of the curability of the curable dental composition, preferred for use as polyol components are C5 to C12 aliphatic diols having a methyl-group side chain, such as 2-methyl-1,4-butanediol, 3-methyl-1,5-pentanediol, 2-methyl-1,8-octanediol, 2,7-dimethyl-1,8-octanediol, 2-methyl-1,9-nonanediol, and 2,8-dimethyl-1,9-nonanediol, more preferably 2-methyl-1,4-butanediol, 3-methyl-1,5-pentanediol, 2-methyl-1,8-octanediol, and 2,7-dimethyl-1,8-octanediol, even more preferably 3-methyl-1,5-pentanediol, and 2-methyl-1,8-octanediol.

The addition reaction between a compound having an isocyanate group and a (meth)acrylic compound having a hydroxyl group may be performed following known methods, and is not particularly limited.

The urethanized (meth)acrylic compound (E-2a) obtained may be the product of a reaction involving any combination of the aforementioned compounds, namely, a polyol having at least one structure selected from the group consisting of a polyester, a polycarbonate, a polyurethane, a polyether, a poly-conjugated diene, and a hydrogenated poly-conjugated diene; a compound having an isocyanate group; and a (meth)acrylic compound having a hydroxyl group.

### <(Meth)Acrylic Compound Having no Urethane Backbone (E-2b)>

Preferably, the (meth)acrylic compound having no urethane backbone (E-2b) comprises a structure (polymer backbone) selected from the group consisting of a polyester, a polycarbonate, a polyurethane, a polyether, a poly-conjugated diene, and a hydrogenated poly-conjugated diene.

In these structures, examples of the polyester include copolymers of dicarboxylic acids (aromatic dicarboxylic acids such as phthalic acid and isophthalic acid, and unsaturated aliphatic dicarboxylic acids such as maleic acid) and aliphatic diols having 2 to 18 carbon atoms, copolymers of dicarboxylic acids (saturated aliphatic dicarboxylic acids such as adipic acid and sebacic acid) and aliphatic diols having 2 to 18 carbon atoms, β-propiolactone polymers, γ-butyrolactone polymers, δ-valerolactone polymers, ε-caprolactone polymers, and copolymers of these. Preferred are copolymers of dicarboxylic acids (aromatic dicarboxylic acids such as phthalic acid and isophthalic acid, and unsaturated aliphatic dicarboxylic acids such as maleic acid) and aliphatic diols having 2 to 12 carbon atoms, and copolymers of dicarboxylic acids (saturated aliphatic dicarboxylic acids such as adipic acid and sebacic acid) and aliphatic diols having 2 to 12 carbon atoms.

Examples of the polycarbonate include polycarbonates derived from C2 to C18 aliphatic diols, polycarbonates derived from bisphenol A, and polycarbonates derived from C2 to C18 aliphatic diols and bisphenol A. Preferred are polycarbonates derived from C2 to C12 aliphatic diols, polycarbonates derived from bisphenol A, and polycarbonates derived from C2 to C12 aliphatic diols and bisphenol A.

Examples of the polyurethane include polymers of C2 to C18 aliphatic diols and C1 to C18 diisocyanates. Preferred are polymers of C2 to C12 aliphatic diols and C1 to C12 diisocyanates.

Examples of the polyether include polyethylene glycol, polypropylene glycol, polybutylene glycol, and poly(1-methylbutylene glycol).

Examples of the poly-conjugated diene and hydrogenated poly-conjugated diene include 1,4-polybutadiene, 1,2-polybutadiene, polyisoprene, poly(butadiene-isoprene), poly(butadiene-styrene), poly(isoprene-styrene), polyfarnesene, and hydrogenated products of these. In view of superior flexibility and water resistance, preferred among these are the polyester, polycarbonate, and poly-conjugated diene structures. Polyols with the aforementioned polymer backbones can be used for the production of (meth)acrylic compound having no urethane backbone (E-2b).

The glass transition temperature and acetone solubility of the (meth)acrylic compound having no urethane backbone (E-2b) can be adjusted by modifying the backbone or molecular weight of a structure (polymer backbone) selected from the group consisting of a polyester, a polycarbonate, a polyurethane, a polyether, a poly-conjugated diene, and a hydrogenated poly-conjugated diene.

Examples of the (meth)acrylic compound having a hydroxyl group include hydroxy (meth)acrylate compounds such as 2-hydroxyethyl (meth)acrylate, 2-hydroxypropyl (meth)acrylate, 2-hydroxybutyl (meth)acrylate, 6-hydroxyhexyl (meth)acrylate, 10-hydroxydecyl (meth)acrylate, 3-chloro-2-hydroxypropyl (meth)acrylate, 2-hydroxy-3-phenoxypropyl (meth)acrylate, glycerin mono(meth)acrylate, 2-hydroxy-3-acryloyloxypropyl (meth)acrylate, 2,2-bis[4-[3-(meth)acryloyloxy-2-hydroxypropoxy]phenyl]propane, 1,2-bis[3-(meth)acryloyloxy-2-hydroxypropoxy]ethane, pentaerythritol tri(meth)acrylate, and tri or tetra(meth)acrylates of dipentaerythritol; and hydroxy(meth)acrylamide compounds such as N-hydroxyethyl(meth)acrylamide, and N,N-bis(2-hydroxyethyl)(meth)acrylamide.

The (meth)acrylic compound having no urethane backbone (E-2b) may be the product of a reaction involving any combination of the aforementioned compounds, namely, a polyol having at least one structure selected from the group consisting of a polyester, a polycarbonate, a polyurethane, a polyether, a poly-conjugated diene, and a hydrogenated poly-conjugated diene; and a (meth)acrylic compound having a hydroxyl group.

In view of the viscosity of the curable dental composition, the (meth)acrylic compound (E-2) has a weight-average molecular weight (Mw) of preferably 1,000 to 80,000, more preferably 2,000 to 50,000, even more preferably 3,000 to 20,000.

In the present invention, the weight-average molecular weight (Mw) means a weight-average molecular weight in terms of polystyrene equivalents, as determined by gel permeation chromatography (GPC).

The weight-average molecular weight of the (meth)acrylic compound (E-2) per (meth)acryl group is preferably 1,250 or more and less than 20,000, more preferably 1,500 or more and 17,500 or less, even more preferably 1,800 or more and 16,000 or less, particularly preferably 2,500 or more and 15,000 or less.

When the number of polymerizable groups in the (meth)acrylic compound (E-2a) falls within the foregoing ranges, crosslinkage takes place in a moderate fashion, and it is possible to maintain mechanical strength in the cured product.

In the (meth)acrylic compound (E-2), the number of polymerizable groups other than (meth)acryl groups is preferably 2 or less, more preferably 0. This is because the presence of polymerizable groups other than (meth)acryl groups (for example, such as vinyl groups, and styrene groups) in the (meth)acrylic compound (E-2) may lead to increased polymerization shrinkage stress depending on the form of polymerization.

In view of the flowability of the curable dental composition, the (meth)acrylic compound (E-2) has a viscosity at 25°C of preferably 1,000 to 10,000,000 mPa·s, more preferably 5,000 to 7,500,000 mPa·s, even more preferably 10,000 to 7,000,000 mPa·s.

In the present invention, the viscosity of (meth)acrylic compound (E-2) means a viscosity measured at 25°C using a Brookfield rotational viscometer. Measurement conditions, such as time and rotational speed, are appropriately adjusted according to the viscosity range.

The (meth)acrylic compound (E-2) may be a commercially available product.

Examples of such commercially available products include urethane polymers having a terminal polymerizable group, such as the Art Resin series (UN-7600, UN-7700) manufactured by Negami Chemical Industrial Co., Ltd.; the Kuraprene series (polyisoprene skeleton or polybutadiene skeleton) manufactured by Kuraray Co., Ltd. (LIR-30, LIR-50, LIR-390, LIR-403, LIR-410, UC-102M, UC-203M, LIR-700, LBR-302, LBR-307, LBR-305, LBR-352, LBR-361, L-SBR-820, L-SBR-841); polyols manufactured by Kuraray Co., Ltd. (P-6010, P-5010, P-4010, P-3010, P-2010, P-1010, F-3010, F2010, F-1010, P-2011, P-1020, P-2020, P-530, P-2030, P-2050, C-2090); and the liquid polybutadiene NISSO-PB manufactured by Nippon Soda Co., Ltd. (B-1000, B-2000, E-2000, BI-2000, BI-3000, G-1000, G-2000, G-3000, GI-1000, GI-2000, GI-3000, TEAI-1000, TE-2000, TE-4000, JP-100, JP-200). Preferred are UN-7600, UN-7700, LBR-302, LBR-307, LBR-305, LBR-352, LBR-361, L-SBR-820, UC-102M, UC-203M, C-2090, P-2020, P-2050, B3000, BI-2000, BI-3000, TEAI-1000, TE-2000, and TE-4000. More preferred are UN-7600, UN-7700, UC-102M, TE-2000, and TE-4000.

### <Hydrophilic Polymer (E-3)>

The hydrophilic polymer (E-3) used in the present invention is preferably one with a hydrophilic group. Preferred examples of the hydrophilic group include carboxyl groups, alkali metal salts of carboxyl groups, sulfonic acid groups, alkali metal salts of sulfonic acid groups, and other functional groups such as hydroxyl groups, amide groups, carbamoyl groups, sulfoneamide groups, and sulfamoyl groups. These groups may occur at any positions within the polymer. Preferred are polymer structures with multiple such hydrophilic groups, bonded to the main chain of the polymer either directly or via a linking group, or bonded to the side chain or graft side chain of the polymer.

For example, the hydrophilic polymer (E-3) can become adsorbed onto the filler (B) through its hydrophilic groups via the adsorption groups of the filler (B) (for example, silanol groups on the surface of silica particles), and form a crosslinked structure with the filler (B) by hydrogen bonding, thereby producing the required unique viscosity behavior.

Examples of the hydrophilic polymer (E-3) include polyvinyl alcohol (PVA), polyvinylpyrrolidone (PVP), polyacrylic acid or salts thereof, and polymethacrylic acid.

Aside from the inorganic fine particle (E-1), (meth)acrylic compound (E-2), and hydrophilic polymer (E-3), any known rheology modifier can be used as rheology modifier (E) without limitation. Examples include cellulose nanofibers, cellulose derivatives, salts of polymethacrylic acid, polysiloxanes and their derivatives (for example, chain polysiloxane compounds, cyclic polysiloxane compounds, cage-like chain polysiloxane compounds), polyamide waxes, polyethylene oxide, organic acid dextrin compounds, dibenzylidene sorbitols, hydrogenated castor oil, ethylene-bis-hydroxystearamide, and natural waxes such as carnauba wax.

These may be used alone, or two or more thereof may be used in combination.

A certain embodiment is, for example, a curable dental composition that does not comprise polysiloxanes or their derivatives.

The rheology modifiers (E) other than inorganic fine particle (E-1) may be used in the same quantities as those used for the inorganic fine particle (E-1).

The content of rheology modifier (E) can be appropriately changed according to the material type so that the time T required for viscosity V3 to reach 10,000 Pa·s upon switching the shear rate from D2 back to D1 falls within the desired range.

A curable dental composition of the present invention may additionally comprise other components such as polymerization inhibitors, ultraviolet absorbers, solvents (for example, water, organic solvents), pH adjusters, colorants, antimicrobial agents, and fragrances, provided that it does not inhibit the effects of the present invention. These may be incorporated alone, or two or more thereof may be used in combination.

Examples of polymerization inhibitors include hydroquinone, hydroquinone monomethyl ether, dibutyl hydroquinone, dibutyl hydroquinone monomethyl ether, t-butylcatechol, 2-t-butyl-4,6-dimethylphenol, 2,6-di-t-butylphenol, and 2,6-di-t-butyl-4-methylphenol.

In certain embodiments, the content of the solvent (for example, water, organic solvents) in the curable dental composition is preferably less than 1 mass%, more preferably less than 0.1 mass%, even more preferably less than 0.01 mass% in total 100 mass% of the curable dental composition.

A curable dental composition of the present invention can be prepared according to an ordinary method, depending on the type and amount of the aforementioned components. Preferably, a curable dental composition of the present invention is used in a two-pack form. The two-pack form can be appropriately selected from various forms, such as powder and liquid, paste and liquid, and two pastes.

In view of handling properties, two pastes are used in more preferred embodiments.

Preferably, in a two-paste curable dental composition, each paste is kept isolated from the other during storage, and the two pastes are kneaded immediately before use to initiate chemical polymerization for curing.

Usually, the pastes are prepared by mixing a liquid component formulated with components other than filler (B), and kneading it with filler (B) (powder).

A certain preferred embodiment is, for example, a two-paste curable dental composition comprising a first agent and a second agent.

Preferably, such a two-paste curable dental composition is a curable dental composition in which the first agent comprises a polymerizable monomer (A), a filler (B), and a chemical polymerization initiator (C-2), and the second agent comprises a polymerizable monomer (A), a filler (B), and a chemical polymerization accelerator (D-2).

Another preferred embodiment is, for example, a curable dental composition in which the first agent comprises a hydroperoxide as chemical polymerization initiator (C-2), and the second agent comprises a polymerizable monomer (A), a filler (B), and, as a chemical polymerization accelerator (D-2), a thiourea compound.

Yet another preferred embodiment is, for example, a curable dental composition in which at least one of the first and second agents in the two-paste curable dental composition comprise a period 4 transition metal compound.

The present invention encompasses embodiments combining the foregoing features in various ways within the technical scope of the present invention, provided that the present invention can exhibit its effects.

### EXAMPLES

The following describes the present invention in greater detail by way of Examples. It should be noted, however, that the present invention is in no way limited by the following Examples.

The components used for the curable dental compositions according to Examples and Comparative Examples are described below, along with the abbreviations.

### [Hydrophilic Polymerizable Monomer (A-1a)]

HEMA: 2-hydroxyethyl methacrylate
#801: 1,2-bis(3-methacryloyloxy-2-hydroxypropoxy)ethane

### [Hydrophobic Polymerizable Monomer (A-1b)]

Bis-GMA: 2,2-bis[4-(2-hydroxy-3-methacryloyloxypropoxy)phenyl]propane
NPG: neopentyl glycol dimethacrylate

### [Filler (B)]

Filler 1: The filler produced in the Production Example 1-1 below was used.
Filler 2: The filler produced in the Production Example 1-2 below was used.

For fillers 1 and 2, the average particle diameter was measured by volume, using a laser diffraction particle size analyzer (Model SALD-2300 manufactured by Shimadzu Corporation) with a 0.2% sodium hexametaphosphate aqueous solution used as dispersion medium.

### [Polymerization Initiator (C)]

### <Photopolymerization Initiator (C-1)>

CQ: camphorquinone

### <Chemical Polymerization Initiator (C-2)>

THP: 1,1,3,3-tetramethylbutylhydroperoxide

### [Polymerization Accelerator (D)]

### <Photopolymerization Accelerator (D-1)>

PDE: N,N-dimethylaminoethyl benzoate

### <Chemical Polymerization Accelerator (D-2)>

DMETU: 4,4-dimethylethylenethiourea
VOAA: vanadylacetylacetonate

### [Rheology Modifier (E)]

### <Spherical Inorganic Fine Particle (E-1a)>

Spherical inorganic fine particle 1: The particles produced in the Production Example 2-1 below were used.

### <Irregularly Shaped Inorganic Fine Particle (E-1b)>

R972: silica (average particle diameter: 16 nm; Aerosil^{®} R972 manufactured by Nippon Aerosil Co., Ltd. under this trade name)
Alumina: average particle diameter: 13 nm; AEROXIDE^{®} Alu C manufactured by Nippon Aerosil Co., Ltd. under this trade name

### <(Meth)Acrylic Compound (E-2)>

UN-7700: urethane acrylate (manufactured by Negami Chemical Industrial Co., Ltd.; weight-average molecular weight (Mw): 15,000 to 25,000, glass transition temperature (Tg): -41°C, polyester backbone, number of polymerizable groups: 2, weight-average molecular weight per acryl group: 7,500 to 12,500)

### [Polymerization Inhibitor]

BHT: 2,6-di-t-butyl-4-methylphenol

### [Production Example 1-1]

### · Production of Filler 1

Eighty parts by mass of distilled water was added to one hundred parts by mass of GM27884 UF2.0 grade (barium glass, manufactured by SCHOTT; average particle diameter: 2.0 µm), and these were mixed at room temperature (about 25°C) to obtain a dispersion. To this dispersion, 3.0 parts by mass of 3-methacryloyloxytrimethoxysilane (KBM-503 manufactured by Shin-Etsu Chemical Co., Ltd. under this trade name) and 200 parts by mass of a 0.3 mass% acetic acid aqueous solution were added in a three-neck flask, and the mixture was stirred at room temperature for 1 hour. After removing water by freeze drying, the resulting material was subjected to heat treatment at 90°C for 3 hours to yield a filler 1 corresponding to irregularly shaped filler (B-2).

### [Production Example 1-2]

### · Production of Filler 2

Two hundred fifty parts by mass of isopropanol was added to one hundred parts by mass of GM27884 UF2.0 grade (barium glass, manufactured by SCHOTT; average particle diameter: 2.0 µm), and these were mixed at room temperature (about 25°C) to obtain a dispersion. To this dispersion, 1.4 parts by mass of 8-methacryloyloxyoctyltrimethoxysilane (KBM-5803 manufactured by Shin-Etsu Chemical Co., Ltd. under this trade name), 5 parts by mass of a 0.3 mass% acetic acid aqueous solution, and 50 parts by mass of isopropanol were added in a three-neck flask, and the mixture was stirred at room temperature for 1 hour. After removing solvent by freeze drying, the resulting material was subjected to heat treatment at 90°C for 3 hours to yield a filler 2 corresponding to irregularly shaped filler (B-2).

### [Production Example 2-1]

### · Production of Spherical Inorganic Fine Particle 1

Sixty parts by mass of isopropanol was added to one hundred parts by mass of silica particles (SNOWTEX^{®} OL manufactured by Nissan Chemical Industries, Ltd. under this trade name; colloidal silica, a water dispersion with a solid concentration of 20 mass%, average particle diameter: 50 nm, average uniformity: 0.8 or more), and these were mixed at room temperature (about 25°C) to obtain a dispersion. To this dispersion, 0.48 parts by mass of 3-methacryloyloxypropyltrimethoxysilane (KBM-503 manufactured by Shin-Etsu Chemical Co., Ltd. under this trade name) and 0.01 parts by mass of a polymerization inhibitor (2,6-di-t-butyl-4-methylphenol (BHT), manufactured by Kanto Kagaku) were added, and these were mixed at 40°C for 72 hours. Subsequently, 0.78 parts by mass of 1,1,1,3,3,3-hexamethyldisilazane (HMDS, manufactured by Shin-Etsu Chemical Co., Ltd.) was added into this mixture, and the resultant mixture was left to stand at 40°C for 72 hours. During this process, the surface treatment of silica particles progressed, causing the silica particles to acquire hydrophobic properties and become unstable in water and isopropanol, resulting in aggregation and precipitation. This was followed by adding 2.6 parts by mass of a 35% hydrochloric acid aqueous solution to the total amount of the mixture resulting from the surface treatment, inducing precipitation of the silica particles. The resulting precipitates were filtered through filter paper (Quantitative Filter Paper No. 5A manufactured by Advantec Co., Ltd.). The resulting residue (solid component) from the filtration process was washed with purified water, and subjected to vacuum drying at 100°C to yield a spherical inorganic fine particle 1.

### [Examples 1-1 to 1-16, 2-1 to 2-11, and Comparative Examples 1-1 to 1-9]

The curable dental compositions were prepared according to the formulations shown in Tables 1 to 3.

The details are as follows.

In the case of rheology modifiers (E) that are in solid form, the components excluding the filler (B) and rheology modifier (E) were mixed at room temperature to form a mixture, and this mixture was mixed with the filler (B) and rheology modifier (E) of Tables 1 to 3 to obtain the dental compositions.

In the case of rheology modifiers (E) that are in liquid form, the components excluding the filler (B) were mixed to form a mixture, and this mixture was mixed with the filler (B) of Tables 1 to 3 to obtain the dental compositions.

### Test Example 1: Viscosity V1 at Shear Rate D1 (0.01 s⁻¹) and Viscosity V2 at Shear Rate D2 (10 s⁻¹)

The viscosity was measured over a total time period of 3 minutes using a rotational rheometer (AR2000 manufactured by TA Instruments Japan Inc. under this trade name), with 1 minute at shear rate D1 (0.01 s⁻¹), followed by 1 minute at shear rate D2 (10 s⁻¹), and then 1 minute at shear rate D1 again. The average viscosity between 30 and 60 seconds from the start of the measurement was denoted as V1, and the average viscosity between 90 and 120 seconds was denoted as V2.

The result for each Example and Comparative Example is presented in the tables below as the mean value for n = 3.

### Test Example 2: Time T Required for Viscosity V3 to Reach 10,000 Pa·s

The viscosity was measured over a total time period of 3 minutes using a rotational rheometer (AR2000 manufactured by TA Instruments Japan Inc. under this trade name), with 1 minute at shear rate D1 (0.01 s⁻¹), followed by 1 minute at shear rate D2 (10 s⁻¹), and then 1 minute at shear rate D1 again. The viscosity after 120 seconds from the start of the measurement was denoted as V3.

Additionally, the time required for viscosity V3 to reach 10,000 Pa·s was determined by subtracting 120 seconds from the time it took for viscosity V3 to reach 10,000 Pa·s from the start of the measurement. This duration was recorded as time T.

The result for each Example and Comparative Example is presented in the tables below as the mean value for n = 3.

### Test Example 3: Film Thickness

The film thickness was determined as an index for evaluating the flowability of the curable dental compositions, in compliance with the method described in ISO4049:2019.

Specifically, the prepared curable dental composition was placed between two glass plates, and a vertical force of 150 ± 2 N was applied. Subsequently, the combined thickness of the two glass plates and the sample film was measured using a micrometer. The difference between this value and the previously measured thickness of the two glass plates was then determined as the film thickness of a dental material for verifying shade match (n = 3). The results are presented in the tables below.

### (Evaluation Criteria for Film Thickness)

A: Less than 20 µm
B: 20 µm or more and 30 µm or less
C: More than 30 µm

### Test Example 4: Removability of Excess Cement

The labial surface of bovine mandibular incisors was polished under running water using silicon carbide paper to expose a flat dentin surface. The exposed flat surface was further polished under running water with #1000 silicon carbide paper, and the surface was dried by removing moisture by air blowing.

After drying, 0.5 g of the curable dental composition from each Example and Comparative Example was applied to the flat surface, and a stainless steel plate, measuring 5 mm × 5 mm, was placed on the composition.

Excess cement that protruded as a result of pressing was irradiated with light for 1 to 2 seconds per side from all four sides of the stainless steel plate, using a dental polymerization photoirradiator PenCure 2000 (manufactured by J. Morita Corp.). The excess resin was then removed with a dental probe, and the removability of the excess cement was evaluated according to the following evaluation criteria. The results are presented in the tables below.

### (Evaluation Criteria for Removability of Excess Cement)

A: Excess cement can be readily and immediately removed following preliminary irradiation
B: Complete curing occurs in parts of excess cement upon preliminary irradiation, but removal is possible with applied force
C: Preliminary irradiation immediately results in complete curing of excess cement, making removal difficult

### Test Example 5: Storage Stability

One milliliter of the curable dental composition was placed in a 5-ml polypropylene tube vial for centrifugation, and centrifuged at 5,000 rpm for 5 minutes using a centrifuge (MCX-150 manufactured by Tomy Seiko Co., Ltd.). The composition was then visually examined to determine whether separation had occurred (n = 1).

Samples showing no visible separation were determined as having good storage stability and marked as "Y", whereas those exhibiting separation were determined as having unfavorable storage stability and marked as "N". The results are presented in the tables below.

### [Examples 3-1 to 3-11]

A two-pack type curable dental composition composed of first and second agents with the formulations shown in Table 4 was used to evaluate each property in the manner described above.

In the evaluation using the two-pack type curable dental composition, the curable dental composition of each Example and Comparative Example used for the Test Examples above was replaced with a curable dental composition prepared by taking the first and second agents of Table 4 in equal amounts and mixing these portions for 10 seconds.

**[Table 1]**

| Components (parts by mass relative to 100 parts by mass of polymerizable monomer (A)) | | Com. Ex. 1-1 | Com. Ex. 1-2 | Com. Ex. 1-3 | Ex. 1-1 | Ex. 1-2 | Ex. 1-3 | Ex. 1-4 | Com. Ex. 1-4 | Ex. 1-5 | Ex. 1-6 | Com. Ex. 1-5 |
|---|---|---|---|---|---|---|---|---|---|---|---|---|
| Hydrophilic polymerizable monomer (A-1a) | HEMA | 2 | 2 | 30 | 2 | 2 | 2 | 2 | 2 | 2 | 2 | 2 |
| Hydrophobic polymerizable monomer (A-1b) | Bis-GMA | 70 | 70 | 70 | 70 | 70 | 70 | 70 | 70 | 70 | 70 | 70 |
| | NPG | 28 | 28 | | 28 | 28 | 28 | 28 | 28 | 28 | 28 | 28 |
| Filler (B) | Filler 1 | 200 | 400 | 200 | 200 | 200 | 200 | 200 | 200 | 200 | 200 | 200 |
| | Filler 2 | | | | | | | | | | | |
| Photopolymerization initiator (C-1) | CQ | 0.1 | 0.1 | 0.1 | 0.1 | 0.1 | 0.1 | 0.1 | 0.1 | 0.1 | 0.1 | 0.1 |
| Photopolymerization accelerator (D-1) | PDE | 0.2 | 0.2 | 0.2 | 0.2 | 0.2 | 0.2 | 0.2 | 0.2 | 0.2 | 0.2 | 0.2 |
| Rheology modifier (E) | Spherical inorganic fine particle 1 | | | | 0.5 | 1 | 10 | 20 | | | | |
| | R972 | | | | | | | | 0.5 | 1 | 10 | 20 |
| | Alumina | | | | | | | | | | | |
| | UN7700 | | | | | | | | | | | |
| Polymerization inhibitor | BHT | 0.1 | 0.1 | 0.1 | 0.1 | 0.1 | 0.1 | 0.1 | 0.1 | 0.1 | 0.1 | 0.1 |
| Viscosity V1 at shear rate D1 (0.01 s⁻¹) [Pa·s] | | 9020 | 19750 | 12830 | 1028 0 | 1278 0 | 1508 0 | 1596 0 | 12920 | 1655 0 | 1805 0 | 19090 |
| Viscosity V2 at shear rate D2 (10 s⁻¹) [Pa·s] | | 11.4 | 320.1 | 89.3 | 11.7 | 32.6 | 45.3 | 56.8 | 33.2 | 44.2 | 61.4 | 115.8 |
| Time T required for viscosity V3 to reach 10,000 Pa·s [s] | | 60 or more | 10 | 10 | 30 | 20 | 20 | 20 | 40 | 20 | 10 | 10 |
| Film thickness | | A | C | A | A | A | A | B | A | A | B | C |
| Removability of excess cement | | C | A | C | B | A | A | A | C | B | A | A |
| Storage stability | | N | Y | N | N | N | Y | Y | N | Y | Y | Y |

**[Table 2]**

| Components (parts by mass relative to 100 parts by mass of polymerizable monomer (A)) | | Com. Ex. 1-6 | Ex. 1-7 | Ex. 1-8 | Com. Ex. 1-7 | Ex. 1-9 | Ex. 1-10 | Ex. 1-11 | Ex. 1-12 | Ex. 1-13 | Com. Ex. 1-8 | Ex. 1-14 | Ex. 1-15 | Com. Ex. 1-9 | Ex. 1-16 |
|---|---|---|---|---|---|---|---|---|---|---|---|---|---|---|---|
| Hydrophilic polymerizable monomer (A-1a) | HEMA | 2 | 2 | 2 | 2 | 2 | 2 | 2 | 2 | 2 | 2 | 2 | 2 | 2 | 2 |
| Hydrophobic polymerizable monomer (A-1b) | Bis-GMA | 70 | 70 | 70 | 70 | 70 | 70 | 70 | 70 | 70 | 70 | 70 | 70 | 70 | 70 |
| | NPG | 28 | 28 | 28 | 28 | 28 | 28 | 28 | 28 | 28 | 28 | 28 | 28 | 28 | 28 |
| Filler (B) | Filler 1 | 200 | 200 | 200 | 200 | 200 | 200 | 200 | 200 | 200 | 200 | 200 | 200 | 200 | 200 |
| | Filler 2 | | | | | | | | | | | | | | |
| Photopolymerization initiator (C-1) | CQ | 0.1 | 0.1 | 0.1 | 0.1 | 0.1 | 0.1 | 0.1 | 0.1 | 0.1 | 0.1 | 0.1 | 0.1 | 0.1 | 0.1 |
| Photopolymerization accelerator (D-1) | PDE | 0.2 | 0.2 | 0.2 | 0.2 | 0.2 | 0.2 | 0.2 | 0.2 | 0.2 | 0.2 | 0.2 | 0.2 | 0.2 | 0.2 |
| Rheology modifier (E) | Spherical inorganic fine particle 1 | | | | | 0.5 | 1 | 10 | 1 | 10 | | | | | |
| | R972 | | | | | | | | | | 0.5 | 1 | 1 | 10 | |
| | Alumina | 0.5 | 1 | 10 | 20 | 0.5 | 1 | 1 | 10 | 10 | 0.5 | 1 | 10 | 10 | |
| | UN7700 | | | | | | | | | | | | | | 3 |
| Polymerization inhibitor | BHT | 0.1 | 0.1 | 0.1 | 0.1 | 0.1 | 0.1 | 0.1 | 0.1 | 0.1 | 0.1 | 0.1 | 0.1 | 0.1 | 0.1 |
| Viscosity V1 at shear rate D1 (0.01 s⁻¹) [Pa·s] | | 16430 | 16780 | 18050 | 20140 | 17750 | 17810 | 16190 | 17110 | 18140 | 15960 | 17520 | 18960 | 21920 | 17700 |
| Viscosity V2 at shear rate D2 (10 s⁻¹) [Pa·s] | | 42.2 | 47.2 | 85.3 | 217.1 | 43.2 | 45.3 | 59.1 | 78.4 | 96.9 | 63.2 | 73.2 | 92.3 | 158.1 | 68.4 |
| Time T required for viscosity V3 to reach 10,000 Pa·s [s] | | 40 | 30 | 20 | 10 | 30 | 20 | 20 | 20 | 10 | 40 | 20 | 10 | 10 | 30 |
| Film thickness | | A | A | B | C | A | A | A | B | B | A | A | A | C | A |
| Removability of excess cement | | C | A | A | A | B | A | A | A | A | C | A | A | A | A |
| Storage stability | | N | Y | Y | Y | N | Y | Y | Y | Y | N | Y | Y | Y | N |

**[Table 3]**

| Components (parts by mass relative to 100 parts by mass of polymerizable monomer (A)) | | Ex. 2-1 | Ex. 2-2 | Ex. 2-3 | Ex. 2-4 | Ex. 2-5 | Ex. 2-6 | Ex. 2-7 | Ex. 2-8 | Ex. 2-9 | Ex. 2-10 | Ex. 2-11 |
|---|---|---|---|---|---|---|---|---|---|---|---|---|
| Hydrophilic polymerizable monomer (A-1a) | HEMA | 2 | 2 | 2 | 2 | 2 | 2 | 2 | 2 | 2 | 2 | 2 |
| Hydrophobic polymerizable monomer (A-1b) | Bis-GMA | 70 | 70 | 70 | 70 | 70 | 70 | 70 | 70 | 70 | 70 | 70 |
| | NPG | 28 | 28 | 28 | 28 | 28 | 28 | 28 | 28 | 28 | 28 | 28 |
| Filler (B) | Filler 1 | | | | | | | | | | | |
| | Filler 2 | 200 | 200 | 200 | 200 | 200 | 200 | 200 | 200 | 200 | 200 | 200 |
| Photopolymerization initiator (C-1) | CQ | 0.1 | 0.1 | 0.1 | 0.1 | 0.1 | 0.1 | 0.1 | 0.1 | 0.1 | 0.1 | 0.1 |
| Photopolymerization accelerator (D-1) | PDE | 0.2 | 0.2 | 0.2 | 0.2 | 0.2 | 0.2 | 0.2 | 0.2 | 0.2 | 0.2 | 0.2 |
| Rheology modifier (E) | Spherical inorganic fine particle 1 | 0.5 | 1 | 10 | 20 | | | 0.5 | 10 | 1 | 10 | |
| | R972 | | | | | 10 | | | | | | 1 |
| | Alumina | | | | | | 10 | 0.5 | 1 | 10 | 10 | 10 |
| | UN7700 | | | | | | | | | | | |
| Polymerization inhibitor | BHT | 0.1 | 0.1 | 0.1 | 0.1 | 0.1 | 0.1 | 0.1 | 0.1 | 0.1 | 0.1 | 0.1 |
| Viscosity V1 at shear rate D1 (0.01 s⁻¹) [Pa·s] | | 9930 | 11740 | 14730 | 15520 | 17840 | 17830 | 17240 | 15800 | 17100 | 16580 | 17820 |
| Viscosity V2 at shear rate D2 (10 s⁻¹) [Pa·s] | | 11.3 | 22.4 | 37.2 | 48.9 | 54.3 | 79.2 | 41.9 | 53.2 | 65.4 | 81.7 | 72.2 |
| Time T required for viscosity V3 to reach 10,000 Pa·s [s] | | 30 | 20 | 20 | 20 | 10 | 20 | 30 | 20 | 20 | 10 | 10 |
| Film thickness | | A | A | A | B | B | B | A | A | B | B | A |
| Removability of excess cement | | B | A | A | A | A | A | B | A | A | A | A |
| Storage stability | | N | N | Y | Y | Y | Y | N | Y | Y | Y | Y |

**[Table 4]**

| Components (parts by mass relative to 100 parts by mass of polymerizable monomer (A)) | | | Ex. 3-1 | Ex. 3-2 | Ex. 3-3 | Ex. 3-4 | Ex. 3-5 | Ex. 3-6 | Ex. 3-7 | Ex. 3-8 | Ex. 3-9 | Ex. 3-10 | Ex. 3-11 |
|---|---|---|---|---|---|---|---|---|---|---|---|---|---|
| First agent | Hydrophilic polymerizable monomer (A-1a) | HEMA | 2 | 2 | 2 | 2 | 2 | 2 | 2 | 2 | 2 | 2 | 2 |
| | Hydrophobic polymerizable monomer (A-1 b) | Bis-GMA | 70 | 70 | 70 | 70 | 70 | 70 | 70 | 70 | 70 | 70 | 70 |
| | | NPG | 28 | 28 | 28 | 28 | 28 | 28 | 28 | 28 | 28 | 28 | 28 |
| | Filler (B) | Filler 1 | | | | | | | | | | | |
| | | Filler 2 | 200 | 200 | 200 | 200 | 200 | 200 | 200 | 200 | 200 | 200 | 200 |
| | Chemical polymerization initiator (C-2) | THP | 4 | 4 | 4 | 4 | 4 | 4 | 4 | 4 | 4 | 4 | 4 |
| | Photopolymerization accelerator (D-1) | PDE | 0.2 | 0.2 | 0.2 | 0.2 | 0.2 | 0.2 | 0.2 | 0.2 | 0.2 | 0.2 | 0.2 |
| | Rheology modifier (E) | Spherical inorganic fine particle 1 | 0.5 | 1 | 10 | 20 | | | 0.5 | 10 | 1 | 10 | |
| | | R972 | | | | | 10 | | | | | | 1 |
| | | Alumina | | | | | | 10 | 0.5 | 1 | 10 | 10 | 10 |
| | | UN7700 | | | | | | | | | | | |
| | Polymerization inhibitor | BHT | 0.1 | 0.1 | 0.1 | 0.1 | 0.1 | 0.1 | 0.1 | 0.1 | 0.1 | 0.1 | 0.1 |
| Second agent | Hydrophilic polymerizable monomer (A-1a) | #801 | 2 | 2 | 2 | 2 | 2 | 2 | 2 | 2 | 2 | 2 | 2 |
| | Hydrophobic polymerizable monomer (A-1 b) | Bis-GMA | 70 | 70 | 70 | 70 | 70 | 70 | 70 | 70 | 70 | 70 | 70 |
| | | NPG | 28 | 28 | 28 | 28 | 28 | 28 | 28 | 28 | 28 | 28 | 28 |
| | Filler (B) | Filler 1 | | | | | | | | | | | |
| | | Filler 2 | 200 | 200 | 200 | 200 | 200 | 200 | 200 | 200 | 200 | 200 | 200 |
| | Photopolymerization initiator (C-1) | CQ | 0.1 | 0.1 | 0.1 | 0.1 | 0.1 | 0.1 | 0.1 | 0.1 | 0.1 | 0.1 | 0.1 |
| | Chemical polymerization accelerator (D-2) | VOAA | 0.1 | 0.1 | 0.1 | 0.1 | 0.1 | 0.1 | 0.1 | 0.1 | 0.1 | 0.1 | 0.1 |
| | | DMETU | 5 | 5 | 5 | 5 | 5 | 5 | 5 | 5 | 5 | 5 | 5 |
| | Rheology modifier (E) | Spherical inorganic fine particle 1 | 0.5 | 1 | 10 | 20 | | | 0.5 | 10 | 1 | 10 | |
| | | R972 | | | | | 10 | | | | | | 1 |
| | | Alumina | | | | | | 10 | 0.5 | 1 | 10 | 10 | 10 |
| | | UN7700 | | | | | | | | | | | |
| | Polymerization inhibitor | BHT | 0.1 | 0.1 | 0.1 | 0.1 | 0.1 | 0.1 | 0.1 | 0.1 | 0.1 | 0.1 | 0.1 |
| Viscosity V1 at shear rate D1 (0.01 s⁻¹) [Pa·s] | | | 10460 | 12100 | 14360 | 15470 | 17370 | 18180 | 17560 | 14290 | 17080 | 17470 | 16440 |
| Viscosity V2 at shear rate D2 (10 s⁻¹) [Pa·s] | | | 10.3 | 23.9 | 39.0 | 45.0 | 58.1 | 82.1 | 41.7 | 50.0 | 59.6 | 87.2 | 68.3 |
| Time T required for viscosity V3 to reach 10,000 Pa·s [s] | | | 30 | 20 | 20 | 20 | 10 | 20 | 30 | 20 | 20 | 10 | 10 |
| Film thickness | | | A | A | A | B | B | B | A | A | B | B | A |
| Removability of excess cement | | | B | A | A | A | A | A | B | A | A | A | A |
| Storage stability | | | N | N | Y | Y | Y | Y | N | Y | Y | Y | Y |

### INDUSTRIAL APPLICABILITY

A curable dental composition according to the present invention can be suitably used as a dental cement in the field of dentistry.

### Reference Signs List

- 1: Dental cement
- 2: Excess cement
- 3: Dental probe

## Claims

1. A curable dental composition comprising a polymerizable monomer (A), a filler (B) with an average particle diameter of 1 µm or more, a polymerization initiator (C), a polymerization accelerator (D), and a rheology modifier (E), wherein:
the polymerization initiator (C) comprises a photopolymerization initiator (C-1),
the curable dental composition exhibits a viscosity V1 of 10,000 Pa·s or more and less than 20,000 Pa·s at an initial shear rate D1 (0.01 s⁻¹) when measured for viscosity over a total time period of 3 minutes using a rotational rheometer, with 1 minute at shear rate D1 (0.01 s⁻¹), followed by 1 minute at shear rate D2 (10 s⁻¹), and then 1 minute at shear rate D1 again,
the curable dental composition exhibits a viscosity V2 of 100 Pa·s or less at shear rate D2 (10 s⁻¹), and
the time T required for viscosity V3 to reach 10,000 Pa·s after the shear rate is switched from D2 back to D1 is less than 40 seconds from when the shear rate is switched from D2 to D1.

2. The curable dental composition according to claim 1, wherein the polymerizable monomer (A) comprises 0 parts or more by mass and less than 15 parts by mass of a hydrophilic polymerizable monomer having no acidic group (A-1a), and 55 parts or more by mass of a hydrophobic polymerizable monomer having no acidic group (A-1b), within total 100 parts by mass of the polymerizable monomer (A).

3. The curable dental composition according to claim 1 or 2, wherein the filler (B) is hydrophobized with a hydrophobizing agent (x-1).

4. The curable dental composition according to claim 1 or 2, wherein the hydrophobizing agent (x-1) comprises a silane coupling agent that has a polymerizable group and is represented by the following general formula (1),
Y-SiRₙX₍₃₋ₙ₎ (1)
wherein Y represents a polymerizable group, or a monovalent organic group having a polymerizable group, R represents a group selected from the group consisting of an alkyl group, an aryl group, and an aralkyl group, X represents a hydroxyl group or a hydrolyzable group, and n represents an integer of 0, 1, or 2, where R may be the same or different from each other when R is plural, and X may be the same or different from each other when X is plural.

5. The curable dental composition according to claim 4, wherein Y in the general formula (1) is a group resulting from the bonding of a (meth)acryloyl group and an organic group via a divalent group containing an oxygen atom, and the organic group is an alkyl group having 5 to 11 carbon atoms.

6. The curable dental composition according to claim 1 or 2, wherein the polymerization initiator (C) comprises a chemical polymerization initiator (C-2).

7. The curable dental composition according to claim 1 or 2, wherein the polymerization accelerator (D) comprises a chemical polymerization accelerator (D-2).

8. The curable dental composition according to claim 7, which comprises a first agent and a second agent, wherein:
the first agent comprises the polymerizable monomer (A), the filler (B), and the chemical polymerization initiator (C-2),
the second agent comprises the polymerizable monomer (A), the filler (B), and the chemical polymerization accelerator (D-2), and
the first agent and/or the second agent comprise the rheology modifier (E).

9. The curable dental composition according to claim 1 or 2, wherein the rheology modifier (E) comprises an inorganic fine particle (E-1) having an average particle diameter of less than 1 µm.

10. The curable dental composition according to claim 9, wherein the inorganic fine particle (E-1) comprises a spherical inorganic fine particle (E-1a).

11. The curable dental composition according to claim 10, wherein the spherical inorganic fine particle (E-1a) is hydrophobized with a hydrophobizing agent (x-1).

12. The curable dental composition according to claim 11, wherein the hydrophobizing agent (x-1) is hydrophobized with a silane coupling agent that has a polymerizable group and is represented by the following general formula (1),
Y-SiRₙX₍₃₋ₙ₎ (1)
wherein Y represents a polymerizable group, or a monovalent organic group having a polymerizable group, R represents a group selected from the group consisting of an alkyl group, an aryl group, and an aralkyl group, X represents a hydroxyl group or a hydrolyzable group, and n represents an integer of 0, 1, or 2, where R may be the same or different from each other when R is plural, and X may be the same or different from each other when X is plural.

13. A dental cement comprising a curable dental composition of claim 1 or 2.
